# EUROPÄISCHE PATENTSCHRIFT

(11) **EP 2 895 458 B1**
(45) Veröffentlichungstag und Bekanntmachung des Hinweises auf die Patenterteilung: **17.08.2016**
(21) Anmeldenummer: 13762114.0
(22) Anmeldetag: 12.09.2013
(51) Int. Cl.: C07C 319/20, C07C 323/58, C07D 251/16

(54) **KONTINUIERLICHES VERFAHREN ZUR HERSTELLUNG VON ORTHO-SUBSTITUIERTEN ANILINEN IN EINEM FLUSSREAKTOR**
CONTINUOUS PROCESS FOR THE PREPARATION OF ORTHO-SUBSTITUTED ANILINES IN A FLOW REACTOR
PROCÉDÉ CONTINU DE FABRICATION D'ANILINES ORTHO-SUBSTITUÉES DANS UN RÉACTEUR D'ÉCOULEMENT

(30) Priorität: 17.09.2012 EP 12184687
(43) Veröffentlichungstag der Anmeldung: 22.07.2015
(73) Patentinhaber: Bayer CropScience AG, 40789 Monheim am Rhein (DE); Bayer Technology Services GmbH, 51368 Leverkusen (DE)
(72) Erfinder: FORD, Mark James, 61389 Schmitten (DE); SEVERINS, Christian, 51379 Leverkusen (DE)
(74) Vertreter: BIP Patents
(86) Internationale Anmeldenummer: PCT/EP2013/068878
(87) Internationale Veröffentlichungsnummer: WO 2014/041052

(56) Entgegenhaltungen:
- WO-A1-96/41799
- WO-A1-2010/127786
- WO-A1-2012/028162
- US-A- 3 972 894
- WRIGHT S W ET AL: "A Convenient Modification of the Gassman Oxindole Synthesis", TETRAHEDRON LETTERS, ELSEVIER, AMSTERDAM, NL, Bd. 37, Nr. 27, 1. Juli 1996 (1996-07-01), Seiten 4631-4634, XP004028978, ISSN: 0040-4039, DOI: 10.1016/0040-4039(96)00920-3 in der Anmeldung erwähnt

## Beschreibung

Die Erfindung betrifft das Gebiet der chemischen Synthese von biologisch aktiven Verbindungen im technischen Maßstab, insbesondere die Synthese von ortho-substituierten Anilinen als Zwischenprodukte für die nachfolgende Herstellung von Feinchemikalien und von Wirkstoffen für die Landwirtschaft, unter Einsatz eines Flussreaktors zur kontinuierlichen Durchführung der Synthese.

Der selektive Austausch von Wasserstoff an einem aromatischen System mit einem substituierten Kohlenstoff-Atom gehört zu einer der fundamentalen Umsetzungen in der organischen Chemie und ist damit bekannt.

Zur Klasse von Verbindungen, die so hergestellt werden können, gehören 2-Oxindole (Dihydroindol-2-one) und deren Vorprodukte. Oxindole und deren Vorprodukte sind vielseitig verwendbare Zwischenprodukte für Wirkstoffsynthesen (Bioorg. Med. Chem. Lett. 2006, 16, 2109; JP 2008-101014; WO 96/41799 A1).

Die meisten der beschriebenen Synthesen von Oxindolen, die sog. Stolle-Synthesen (siehe Schema 1 (a)), verwenden eine Variation der Friedel-Crafts-Reaktion (Stolle Synthesis, W.C. Sumpter, Chem. Rev. 1945, 37, 443-449). Jedoch sind Stolle-Synthesen nur bedingt einsetzbar, da deren Durchführung stark saure Bedingungen sowie den Einsatz eines elektronenreichen Anilins erfordern.

Daneben sind Radikal- (siehe Schema 1 (b)), Nitrenium-ion-Reaktionen und Organolithium-Reaktionen sowie photochemische Methoden bekannt. Deren Anwendbarkeit ist jedoch ebenfalls limitiert durch den jeweils herzustellenden Typ von Oxindolen, die Kompatibilität von Substraten, den Reaktionsbedingungen sowie durch die Bedingung, dass der Aromat bereits einen substituierbaren Halogen-Substituenten aufweisen muss.

Radikal-Verfahren (siehe Schema 1(b)) sind beschrieben in: Zard et al., Tetrahedron Lett. 1994, 35, 9553-9556; Zard et al., Tetrahedron Lett. 1994, 35, 1719-1722; Jones et al., Tetrahedron Lett. 1994, 35, 7673-7676; Kikugawa et al., Chem. Letters 1987, 1771-1774; Clark etal., Synthesis 1991, 871-878; Yonemitsu et. al., Chem. Pharm. Bull. 1981, 29, 128-136.

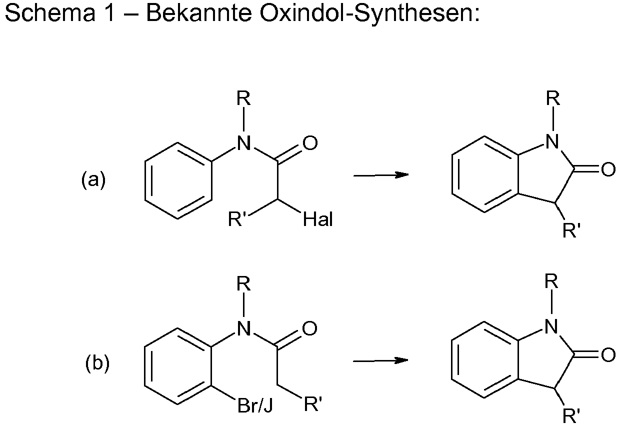

Das Verfahren von Gassman et al. (Organic Synthesis Coll., Vol. 6, 601 und Vol. 56, 72), welches, ausgehend von Anilin und Methylthioacetat-Ester, über Chlorierung und Behandlung mit Triethylamin bei -70°C erfolgt (s. Schema 2), erscheint geeignet im Hinblick auf die Durchführbarkeit, die Verfügbarkeit der Edukte, die Dauer der Reaktion (Reaktionsgeschwindigkeit) und die Reproduzierbarkeit.

Jedoch ist bekannt, dass gute Ausbeuten nur bei tiefen Temperaturen erzielt werden können, nämlich, wenn die während der Reaktion auftretenden instabilen N-Chlor- (1) oder N-Sulfonium- (2) Zwischenprodukte (siehe Schema 2) unter -65°C, nämlich im Normalfall bei -78°C, gebildet werden (Gassman et. al., J. Am. Chem. Soc., 1974, 96(17), 5508; Gassman et al., J. Am. Chem. Soc., 1974, 96(17), 5512; WO 96/41799 A1). Die Durchführung einer Reaktion im technischen Maßstab bei Temperaturen unter -65°C erfordert jedoch bereits apparativ einen höheren Aufwand und ist zudem aufgrund der durch die Kühlung verursachten hohen Betriebskosten nachteilig. 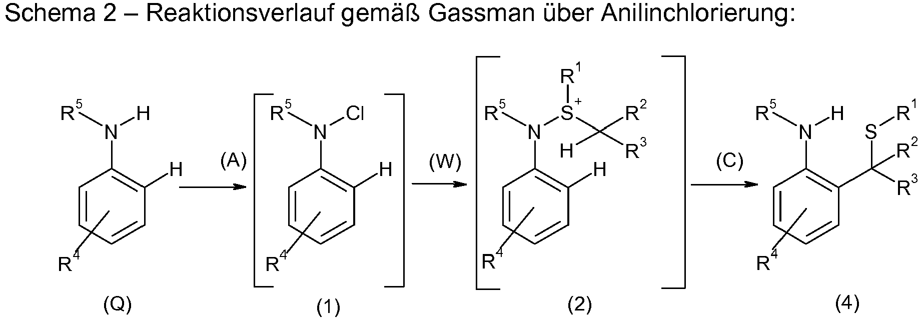
Q = Anilin
A = Chlorierungsmittel (z.B.: tert-Butylhypochlorit, t-BuOCl)
W = Thioether (R¹-S-CHR²R³)
C = Tertiäre Amin-Base (z.B.: Triethylamin)

Chlorierungsmittel der Wahl ist laut Literatur das instabile und explosive tert-Butylhypochlorit, wobei das Nebenprodukt der Chlorierung den neutralen tert-Butylalkohol ergibt. In den wenigen Fällen, bei denen Sulfurylchlorid (SO₂Cl₂) als Chlorierungsmittel eingesetzt worden war, wurde eine zweite, nicht nukleophile Base, wie "proton sponge", eingesetzt (Johnson, J. Org. Chem. 1990, 55, 1374; Warpehoski, Tetrahedron Lett. 1986, 27, 4103). Da beide Varianten bei tiefen Temperaturen durchgeführt werden müssen, ist dies jedoch keine vorteilhafte Lösung zur Durchführung der Reaktion im technischen Maßstab. Die Zugabe der tertiären Aminbase (C) erfolgt beim Gassman-Verfahren erst im abschließenden Schritt (siehe Schema 2) und dient der Deprotonierung des Zwischenproduktes (2) zur Einleitung der Umwandlung des Zwischenprodukts (2) in das ortho-substituierte Anilin (4).

Aus WO 2012/028162 A1 ist ein verbessertes Batch-Verfahren zur Herstellung von Verbindungen der Formel (4) ebenfalls ausgehend von einem Thioether und einem Anilin der Formel (Q) bekannt, wobei als Chlorierungsmittel ebenfalls Sulfonylchlorid (SO₂Cl₂) eingesetzt wird.

Der Kern der in WO2012/028162 A1 offenbarten Lehre betrifft die Erkenntnis, dass ein Überschuss an den überraschenderweise elektronenarmen Anilinen als milde Base bei der Bildung von Produkt der Formel (4) fungiert. Dies ist überraschend gegenüber der Standard-Gassmann-Reaktion, welche die abschließende Zugabe eines zusätzlichen tertiären Amins lehrt (vgl. Schemata 2 und 3: C = Tertiäre Amin-Base, z.B.: Triethylamin). Das Anilin ist offensichtlich in der Lage, die Umlagerung zum Produkt der Formel (4) zu katalysieren und daher auch HCl von einem Chlorsulfonium-Zwischenprodukt der Formel (3) zu eliminieren, welches nur zu Nebenreaktionen führen würde. Hinweise auf besondere Maßnahmen zur Durchführung der in WO 2012/028162 A1 offenbarten Reaktion unter Einsatz eines Flussreaktors zur kontinuierlichen Durchführung der Synthese werden in dem genannten Dokument nicht gegeben.

Aus US 3,972,894 ist ein weiteres von Gassmann entwickeltes Batch-Verfahren bekannt, bei dem zur Herstellung von Oxindolen zunächst ortho-substituierte Aniline als Intermediate hergestellt werden. Als Edukte zur Gewinnung der ortho-substituierten Aniline werden N-Halogenaniline und β-Thioester oder β-Thioamide eingesetzt. Bei der Umsetzung der Edukte wird als Zwischenprodukt ebenfalls eine Azasulfonium-Verbindung der Formel (2) gebildet, welche ebenfalls erst im abschließenden Schritt (siehe Schema 2) mit einer Base zu einem ortho-substituierten Anilin umgesetzt wird. Als geeignete Basen sind niederkettige Alkylamine, wie Ethylamin, Diethylamin, Triethylamin, Tributylamin und aromatische Amine wie z.B. Pyridin genannt. Hinweise auf besondere Maßnahmen zur Durchführung der in US 3,972,894 offenbarten Reaktion unter Einsatz eines Flussreaktors zur kontinuierlichen Durchführung der Synthese werden in dem genannten Dokument nicht gegeben.

Für die Synthese von Oxindolen ist das durch die Anwendung der Gassman Reaktion erhaltene Produkt (4) eine Schlüsselvorstufe. Eine neue Alternative zur weiteren Umsetzung von Verbindungen (4) zum jeweiligen Oxindol ist in WO 2010/127786 A1 beschrieben.

Eine weitere Alternative zur Herstellung von ortho-subsitituierten Anilinen der Formel (4) beschreibt Wright et al. (Tetrahedron Lett. 1996, 37, 4631). Dabei wird das Chlorsulfonium-Zwischenprodukt (3) aus einem Sulfoxid und Oxalylchlorid hergestellt und weiter zu dem Produkt der Formel (4) umgesetzt (s. Schema 3). Die Zugabe der tertiären Aminbase (C) erfolgt bei dem von Wright et al. beschriebenen Verfahren erst im abschließenden Schritt (siehe Schema 3).

Jedoch ist das Chlorsulfonium-Zwischenprodukt (3) ebenfalls instabil. Außerdem muss für diese Reaktion zuerst das Sulfoxid hergestellt und isoliert werden. Aus Gründen der Stabilität muss die Reaktion ebenfalls bei tiefen Temperaturen, nämlich bei -78°C ablaufen. Zudem muss die Reaktion stufenweise durchgeführt werden, um eine Reaktion zwischen Anilin und Oxalylchlorid zu vermeiden. 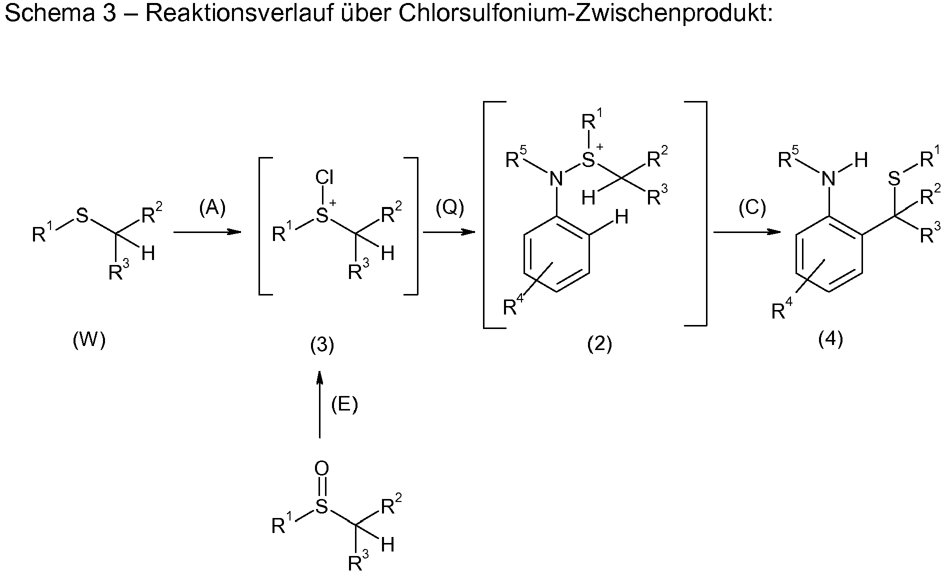
W = Thioether (R¹-S-CHR²R³)
A = Chlorierungsmittel (z.B.: tert-Butylhypochlorit, t-BuOCl)
Q = Anilin
C = Tertiäre Amin-Base (z.B.: Triethylamine)
E = Oxalylchlorid (COCl)₂

Die Gründe für die Empfindlichkeit der in Schema 3 gezeigten Reaktion gegenüber höheren Reaktionstemperaturen, d.h. gegenüber Reaktionstemperaturen von über -70°C, und die Gründe für die Notwendigkeit die Reaktion immer stufenweise durchzuführen, sind vielfältig.

Wesentlich ist zunächst, dass die an der in Schema 3 gezeigten Reaktion beteiligten funktionellen Gruppen, d.h. das Stickstoff-Atom des Anilins und das Schwefel-Atom des Thioethers, unverändert sowohl im Produkt (4) als auch im Edukt vorkommen.

Vor diesem Hintergrund wäre eine selektive Chlorierung, in der das Produkt (4) direkt, d.h. während der Reaktion, und zugleich mit hoher Ausbeute, d.h. quantitativ, gebildet wird, nicht zu erwarten. Dies erklärt auch warum alle literaturbekannten Methoden eine stufenweise Reaktionsführung anwenden, bzw. vorschlagen.

Außerdem besteht bei der Chlorierung von Verbindungen wie Anilinen das Problem der Kernchlorierung, d.h. der unerwünschten Chlorierung des aromatischen Benzolkerns anstatt der gewünschten Chlorierung des Aminosubstituenten. Durch Kernchlorierung kann sich das N-Chloranilin bei Reaktionstemperaturen von über -65°C in einen im Kern chlorierten Aromaten umwandeln (siehe Schema 4).

Das Problem der Kernchlorierung lässt sich gemäß Lengyel et al. am Beispiel von Acetanilid verdeutlichen. Die Wahrscheinlichkeit der Kernchlorierung ist davon abhängig, ob der Benzolkern elektronenreich oder eher elektronenarm ist. Obwohl Acetanilid im Vergleich zu N-Chloranilin deutlich weniger elektronenreich ist und somit deutlich weniger zur Kern-Chlorierung neigen sollte, erfolgt mit tert-Butylhypochlorit als Chlorierungsmittel bereits bei einer Reaktionstemperatur von 0°C Kernchlorierung (Lengyel et al. Synth. Comm., 1998, 28 (10), 1891-1896).

Weiterhin können die Sulfonium-Zwischenprodukte (2) oder (3) in Gegenwart von Basen durch Eliminierung das reaktive Nebenprodukt (5) bilden. Das reaktive Nebenprodukt (5) kann z.B. mit einem Anilin kondensieren. Dabei wurde durch die sog. Pummerer-Oxidation des R²-CH-R³-Restes irreversibel die Nebenkomponente (6) erzeugt (s. Schema 4). Zudem können sich auch andere Oxidationsprodukte (Dimere) bilden.

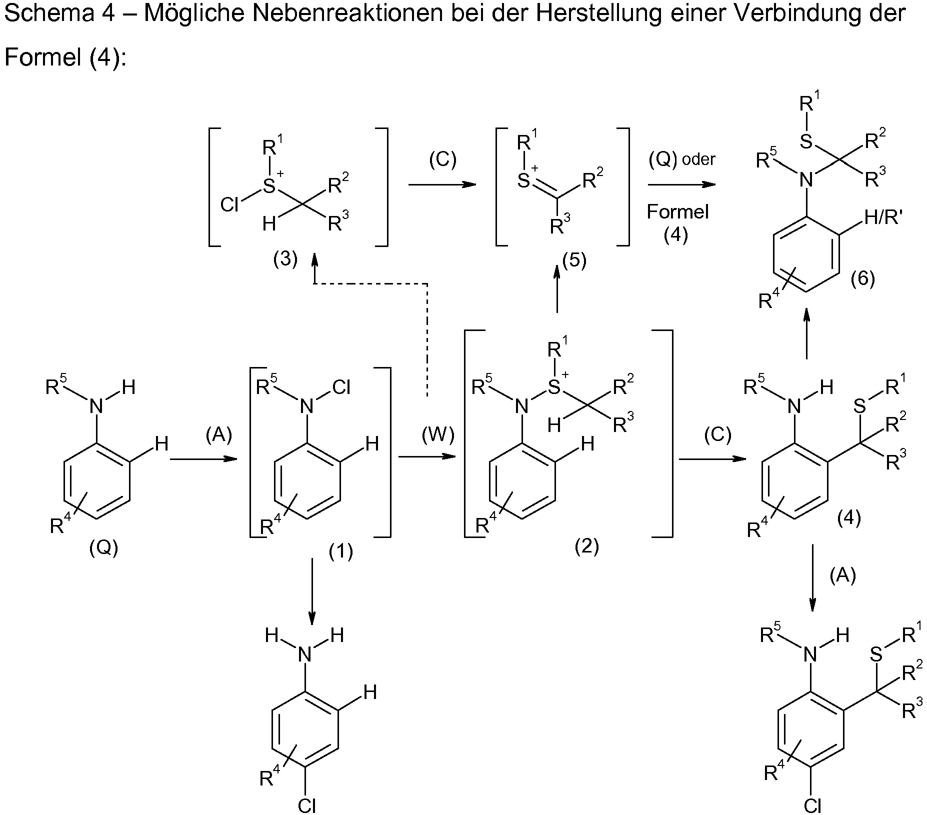

### (Die Bezeichnungen der Edukte und der übrigen Reagenzien in Schema 4 entsprechen den Bezeichnungen aus Schema 3.)

In WO 2010/127786 A1 ist beschrieben wie einige der vorgenannten Nachteile überwunden werden können und die Ausführbarkeit einer technischen Batch-Reaktion durch Beispiele belegt. Bei Durchführung einer Batch-Reaktion gemäß WO 2010/127786 A1 werden jedoch Temperaturen unter -20°C als erforderlich angesehen, so dass bei der Durchführung der Batch-Reaktion im technischen Maßstab hohe Energiekosten entstehen. Außerdem erfordert die wiederholt erforderliche Kühlung des Batch-Dessels ausgehend von der Raumtemperatur auf unter -20°C viel Zeit.

Vor diesem Hintergrund wäre es von erheblichem Vorteil, wenn die Reaktion zur Herstellung von Verbindungen der Formel (4) in einem Flussreaktor durchgeführt werden könnte.

In einem Flussreaktor muss ein im Vergleich zur Batch-Reaktion kleines Volumen auf niedrige Temperatur gebracht werden, wobei die weitere Reaktion kontinuierlich, d.h. ohne Unterbrechung, durchgeführt werden kann. Die Vorteile des Einsatzes eines Flussreaktors bestehen, allgemein betrachtet, in der Verbesserung der Produktivität bei vereinfachtem Verfahrensablauf.

Allerdings erwies sich die Übertragung der in WO 2010/127786 A1 offenbarten, für die Durchführung einer Batch-Reaktion vorgesehenen, Reaktionsbedingungen, auf die Bedingungen in einem Flussreaktor, d.h. die Übertragung der Bedingungen auf die kontinuierliche Durchführung der Reaktion in einem Flussreaktor, bereits aufgrund der Bildung von schwer löslichen Salzen im Reaktionsgemisch als problematisch und nicht umsetzbar.

Tatsächlich sind Reaktionen, bei denen Salze gebildet werden, sehr schwer in Flussreaktoren zu bewältigen, wenn diese Salze als Feststoffe ausfallen. Solche Feststoffe verursachen in einem Flussreaktor die Verkrustung und Blockade der Mikrokanäle und kleinvolumigen Mischkammern. Allerdings beruht die Funktion eines Flussreaktor-Systems gerade auf der Gängigkeit dieser Mikrokanäle und Mischkammern. Die während der Reaktion gebildeten Ausfällungen und die daraus entstehenden Suspensionen sind daher unbedingt zu vermeiden, wenn eine Reaktion in einem Flußreaktor ohne Unterbrechung und auf verlässliche Weise durchführbar sein soll. Besonders ungünstig ist auch das Auftreten von schwer löslichen Salzen und/oder von viskosen Suspensionen, wenn eine Reaktion mit einer technisch relevanten Konzentration, d.h. im technischen Maßstab, ausgeführt werden soll.

Die Anwendung der in WO 2010/127786 A1 offenbarten Bedingungen, die allein auf die Durchführung des sog. Batch-Verfahrens ausgerichtet sind, bei der kontinuierlichen Durchführung der Reaktion in einem Flussreaktor erwies sich als sehr problematisch.

Die Schwierigkeiten könnten darauf beruhen, dass das bei dem vorliegenden Verfahren als Edukt eingesetzte Anilin während der Reaktion aufgrund seiner basischen Eigenschaften in Gegenwart eines Chlorierungsmittels chloriert wird. Bei der Chlorierung entsteht HCl, in dessen Gegenwart jedoch zumindest ein Teil des chlorierten Anilins als festes HCl-Salz ausfällt.

Bei einer Batch-Reaktion sind diese Ausfällungen, im Gegensatz zur Durchführung derselben Reaktion im Flussreaktor, nicht weiter störend, weil das HCl-Salz des Anilins mit dem freien Anilin im Gleichgewicht steht. Das gilt auch dann, wenn die Einstellung des Gleichgewichts wegen der Löslichkeit des Salzes nur langsam erfolgt. Bei einer Batch-Reaktion kann sich das genannte Gleichgewicht, bei fortschreitender Chlorierung immer wieder erneut einstellen, so dass trotz abnehmender Menge an freiem Anilin, wiederholt neues Anilin für die Reaktion zur Verfügung steht.

Bei Durchführung der gleichen Reaktion in einem Flussreaktor sind dagegen die Rahmenbedingungen für die Neueinstellung des Gleichgewichts nicht gegeben. Hauptgrund dafür, ist die vergleichsweise kurze Verweildauer der Edukte in dem jeweiligen Reaktorabschnitt, der für den Ablauf der Reaktion im Flussreaktor vorgesehen ist. Daher kann die Einstellung eines chemischen Gleichgewichtes im jeweiligen Abschnitt eines Flussreaktors nicht, bzw. nur sehr bedingt erfolgen.

Bei der Übertragung der für die Batch-Reaktion etablierten Bedingungen auf die Durchführung derselben Reaktion in einem Flussreaktor fällt das Edukt als Anilin-HCl-Salz aus. Aufgrund dieser Salzbildung entsteht eine dicke, d.h. hoch viskose, Suspension. Diese Suspension ist nicht forderfähig, so dass die Passage des Reaktionsgemischs durch die den Flussreaktor aufbauenden Komponenten entweder sehr erschwert oder unmöglich ist.

Außerdem wird der Reaktion durch die Salzbildung das Edukt Anilin entzogen, so dass sich im nächsten Reaktionsabschnitt des Flussreaktors ein Überschuss an Chlorierungsmittel ergibt. Dieser Überschuss erhöht die Wahrscheinlichkeit, dass unerwünschte Nebenreaktionen erfolgen, insbesondere die unerwünschte Chlorierung des ebenfalls als Edukt eingesetzten Thioethers.

Vor diesem Hintergrund besteht die Aufgabe der Erfindung in der Bereitstellung eines modifizierten Verfahrens, das die Herstellung von ortho-substituierten Anilinen der Formel (4) ausgehend von Anilinen der Formel (Q) im technischen Maßstab unter Einsatz eines Flussreaktors ermöglicht. Insbesondere besteht die Aufgabe der Erfindung in der Bereitstellung eines kontinuierlichen Verfahrens zur Herstellung von ortho-substituierten Anilinen der Formel (4) unter Vermeidung von viskosen feststoffhaltigen Suspensionen während der Durchführung der Reaktion in einem Flussreaktor.

Überraschend wurde im Rahmen der anfänglichen Bemühungen zur Lösung der Aufgabe erkannt, dass die bereits zum Beginn der Reaktion erfolgende Zugabe mindestens einer Stickstoff-Base, die keine NH-Gruppe aufweist, die Löslichkeit des Reaktionsgemischs insgesamt verbessert.

Gegenstand der Erfindung ist somit ein Verfahren zur kontinuierlichen Herstellung von Verbindungen der Formel (4) 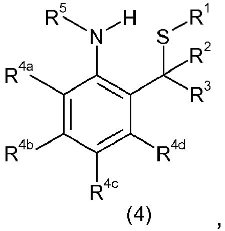 worin
R¹ für (C₁-C₆) Alkyl, substituiertes (C₁-C₆) Alkyl, Aryl oder substituiertes Aryl steht,
R² für einen elektronenziehenden oder für einen aktivierenden Substituenten steht, der ausgewählt ist aus der Gruppe bestehend aus
   - CN,
   - NO₂,
   - CO-R^{1'}, wobei R^{1'} wie R¹ definiert ist und R^{1'} gleich oder anders ist als R¹,
   - CO-X, wobei X für OR^{1"}, SR^{1"} oder NR^{2'}R^{2"} steht, worin R^{1"} wie R¹ definiert ist und R^{1"} gleich oder anders ist als R¹, und worin R^{2'} und R^{2"} unabhängig voneinander jeweils für H, (C₁-C₆) Alkyl, substituiertes (C₁-C₆) Alkyl, Aryl oder substituiertes Aryl stehen oder R^{2'} und R^{2"} alternativ einen Ring bilden,
   - SO(n)-R^{1"'}, worin R^{1"'} wie R¹ definiert ist, wobei R^{1"'} jeweils gleich oder anders ist als R¹ und wobei n 0, 1, oder 2 ist,
   - Aryl, und
   - Heteroaryl,
R³ für H, (C₁-C₆) Alkyl, substituiertes (C₁-C₆) Alkyl, Aryl oder substituiertes Aryl steht,
R^{4a} bis R^{4d} unabhängig voneinander ausgewählt sind aus der Gruppe bestehend aus Wasserstoff, Fluor, Chlor, Brom, Iod, CN, NO₂ sowie aus
   (C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   (C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
   (C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   (C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
   CO-X, wobei X für OR^{1"'}, SR^{1"'} oder NR^{2'}R^{2"} steht, worin R^{1"} wie R¹ definiert ist und R^{1"} gleich oder anders ist als R¹, und worin R^{2'} und R² wie R³ definiert sind, wobei R^{2'} und R² jeweils gleich oder anders sind als R³, oder R^{2'} und R^{2"} einen Ring bilden,
   Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R⁵ für H, (C₁-C₆) Alkyl, substituiertes (C₁-C₆) Alkyl, Aryl oder substituiertes Aryl steht,
bei dem in einem Flussreaktor in Gegenwart eines organischen Lösungsmittels als Ed u kte
- ein Anilin (Q) 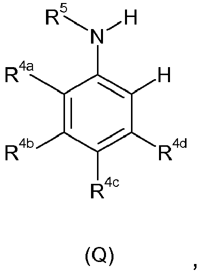 worin
   R^{4a} bis R^{4d} und R⁵ wie für Verbindungen der Formel (4) definiert sind, und
- ein Thioether (W) 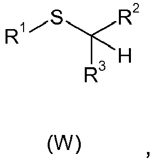 worin
   R¹, R² und R³ wie für Verbindungen der Formel (4) definiert sind, eingesetzt werden, wobei die Edukte der Formeln (Q) und (W) in Gegenwart
      - eines Chlorierungsmittels, und
      - mindestens einer Stickstoff-Base, die keine NH-Gruppe aufweist, umgesetzt werden.

Bevorzugt sind Verbindungen der Formel (4), in welchen die Reste R^{4a} bis R^{4d} unabhängig voneinander jeweils für H, F, Cl, Br, I, CF₃, CN, NO₂ oder CO-X stehen, wobei X für OR^{1"}, SR^{1"} oder NR^{2'}R^{2"} steht, worin R^{1"} wie R¹ definiert ist und R^{1"} gleich oder anders ist als R¹, und worin R^{2'} und R^{2"} unabhängig voneinander jeweils für H, (C₁-C₆) Alkyl, substituiertes (C₁-C₆) Alkyl, Aryl oder substituiertes Aryl stehen. Die Reste R^{2'} und R^{2"} können alternativ auch einen Ring bilden.

Ausgehend von Verbindungen der Formel (4) werden Oxindol-Verbindungen der Formel (7-1) 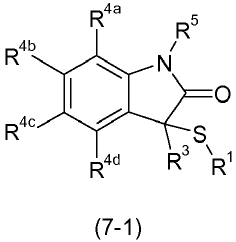 erhalten durch die Extraktion des tertiären Amins aus der die Verbindungen der Formel (4) enthaltenden Reaktionslösung oder durch das Aufkonzentrieren der die Verbindungen der Formel (4) enthaltenden Reaktionslösung und anschließendes Versetzen des Konzentrats mit einer alkoholischen HCl-Lösung, bevorzugt 0,4 N HCl, wobei bei einer Temperatur im Bereich von 0° bis 40°C nachgerührt wird.

Das erfindungsgemäße Verfahren zeichnet sich dadurch aus, dass dem Reaktionsgemisch von Anfang an eine Stickstoff-Base, die keine NH-Gruppe aufweist, zugegeben wird. Durch die Zugabe der Stickstoffbase wird das Ausfallen von bereits chlorierten Anilinmolekülen als HCl-Salz unterbunden.

Die bereits bei Reaktionsbeginn, und optional im Verlauf der Reaktion gegebenenfalls wiederholt, zugegebene Stickstoffbase zeichnet sich dadurch aus, dass es keinen an den Stickstoff gebundenen Wasserstoff, d.h. keine NH-Gruppe, aufweist. Stickstoff-Basen, die keine NH-Gruppe aufweisen, können auch als "Amine ohne freies NH" bezeichnet werden. Eine große Gruppe von Stickstoff-Basen, die keine NH-Gruppe aufweisen, sind tertiäre Amine. Die für die Durchführung des erfindungsgemäßen Verfahrens einsetzbaren Amine zeichnen sich außerdem durch ihre basischen Eigenschaften aus.

Neben tertiären Aminen sind auch unsubstituierte Pyridine oder substituierte oder unsubstituierte Chinoline Stickstoff-Basen, die jeweils ebenfalls keine NH-Gruppe aufweisen und zur Durchführung des erfindungsgemäßen Verfahrens geeignet sind.

Der Kern der Erfindung betrifft den unerwarteten und zugleich sehr vorteilhaften Umstand, dass das in Gegenwart eines Chlorierungsmittels entstehende HCl-Salz einer Stickstoff-Base ohne NH-Gruppe, insbesondere das in Gegenwart eines Chlorierungsmittels entstehende HCl-Salz einer der im vorhergehenden Absatz genannten bevorzugten Stickstoffbasen, in dem zur Herstellung von Verbindungen der Formel (4) gewählten Lösungsmittel während der gesamten Reaktion gelöst vorliegt.

Der Vorteil beruht darauf, dass die Stickstoffbasen das in Gegenwart des Chlorierungsmittels entstehende HCl aufnehmen, ohne selbst chloriert zu werden, und dabei in dem für die Durchführung der Reaktion zur Herstellung von Verbindungen der Formel (4) gewählten Lösungsmittel gelöst bleiben. So wird durch die Zugabe einer der genannten Stickstoff-Basen erreicht, dass das Edukt Anilin nicht als unlösliches HCl-Salz ausfällt.

Der Einsatz von Stickstoff-Basen, die jeweils keine NH-Gruppe aufweisen, bewirkt auch die Vermeidung anderer Nebenreaktionen. So kann in Gegenwart eines Chlorierungsmittels bei einer Aminbase mit "freiem H", wie z.B. bei einem sekundären Amin, eine N-Cl Bindung entstehen. Das chlorierte Amin kann in einer unerwünschten Nebenreaktion mit dem Thioether (W) reagieren, so dass der angestrebten Hauptreaktion der Thioether als Edukt entzogen wird.

Zudem bewirkt der Aufbau des nach Vermeidung von schwer löslichen oder von viskosen Reaktionsgemischen einsetzbaren Flussreaktors eine schnelle räumliche Trennung von Edukten und Produkten. Durch die schnelle räumliche Trennung der Edukte von den Produkten in einem Flussreaktor kann die erneute Chlorierung des bereits chlorierten Reaktionsprodukts der Formel (4) zusätzlich vermieden werden.

Ungeachtet des Problems der Bildung von als Feststoff ausfallenden Anilin-HCl-Salzen während einer chemischen Reaktion, ist im Hinblick auf das vorliegende Verfahren zunächst zu bedenken, dass man nach bisherigem Kenntnisstand bei einer Reaktion gemäß Schema 2, zur Vermeidung von Nebenreaktionen (siehe Schema 4) nicht erwägen würde, dem Reaktionsgemisch vor Abschluss der Chlorierung, ein tertiäres Amin zuzugeben.
Weiterhin ist im Hinblick auf die Abgrenzung der vorliegenden Erfindung gegenüber dem bekannten (originalen) Gassman-Verfahren (siehe Schema 2) festzustellen, dass beim originalen Gassman-Verfahren die Bildung der Verbindung (2) bereits abgeschlossen ist, bevor dem Reaktionsgemisch ein tertiäres Amin zugegeben wird. Beim originalen Gassman-Verfahren erfolgt die Zugabe des tertiären Amins allein, um mittels Deprotonierung die Umwandlung von Verbindungen der Formel (2) in die Verbindungen der Formel (4) zu katalysieren.

Der Erwartung nach müsste die Zugabe eines tertiären Amins bereits zum Reaktionsbeginn bei einer Reaktion gemäß Schema 2 zu einer unerwünschten Chlorierung des tertiären Amins führen. Bei dem durch die Chlorierung entstandenen Chlorsalz des tertiären Amins handelt es sich um eine nicht inerte, d.h. um eine reaktive, Spezies. Diese reaktive Spezies müsste der Erwartung nach unerwünschte Nebenreaktionen, wie beispielsweise die Chlorierung oder Aminierung des als Edukt eingesetzten Thioethers bewirken. Vermutlich aus diesen Gründen ist bei einer Reaktion gemäß Schema 2 die Zugabe einer Stickstoffbase ohne NH-Gruppe bereits zu Reaktionsbeginn bislang nicht bekannt.

Das vorliegende Verfahren zur kontinuierlichen Herstellung eines ortho-substituierten Anilins der Formel (4) in einem Flussreaktor in Gegenwart einer Stickstoff-Base, die keine NH-Gruppe aufweist, ist mit dem wesentlichen Vorteil verbunden, dass das HCl-Salz der eingesetzten Stickstoff-Base in dem für die Reaktion gewählten organischen Lösungsmittel während der gesamten Reaktion, d.h. bei den in den jeweiligen Komponenten des Flussreaktors herrschenden unterschiedlichen Temperaturen, gelöst vorliegt.

Ein wesentlicher die Erfindung betreffender Aspekt ist somit, neben der Auswahl einer geeigneten Stickstoff-Base, die Beachtung und vorherige Bewertung der Löslichkeit des HCl-Salzes der Stickstoff-Base, d.h. die Bewertung der Löslichkeit des Salzes, das sich aus der jeweils ausgewählten Stickstoff-Base in Gegenwart des bei der Reaktion eingesetzten Chlorierungsmittels bildet.

Löslichkeit und Mischbarkeit der Edukte sind wesentliche Voraussetzungen für die Umsetzung der Edukte in einem Flussreaktor, dass dieser auf einer kontinuierlichen Reaktionsführung basiert. Aufgrund der Löslichkeit des HCl-Salzes der Stickstoff-Base können das Anilin (Q), das Chlorierungsmittel sowie der Thioether (W) schnell miteinander und/oder nacheinander gemischt und direkt zu Verbindungen der Formel (4) umgesetzt werden.

Der selektive Verlauf der Reaktion, die das Produkt zugleich auch in hoher Reinheit ergibt, ist besonders überraschend, weil die Stickstoff-Base bei der Chlorierung mit dem Anilin um das Chloratom konkurriert. Die chlorierte Stickstoffbase kann in einer unerwünschten Nebenreaktion mit Thioether reagieren. In diesem ungünstigen, aber zugleich wahrscheinlichen, Fall stünde der Thioether nicht mehr als Reaktionspartner für das Anilin zur Verfügung. Dabei kann nicht vorhergesagt werden, auch nicht von einem Fachmann, ob, bzw. in welchem Umfang, die Zugabe des tertiären Amins (P) die Selektivität der Reaktion zwischen den Edukten, d.h. zwischen dem Anilin (Q) und dem Thioether (W), nach vorangegangener Zugabe eines Chlorierungsmittels negativ beeinflusst.

Die Reaktionstemperatur bei Durchführung der Reaktion im Flussreaktor liegt bevorzugt zwischen - 65° C und 0°C. Dabei ist es besonders bevorzugt wenn die Reaktionstemperatur zwischen - 55°C und - 10°C liegt. Am meisten bevorzugt ist eine Reaktionstemperatur zwischen - 45°C und - 20°C.

Eine bevorzugte Gruppe von Stickstoffbasen ohne NH-Gruppe (P) bildet die Gruppe der tertiären Amine. Besonders bevorzugte tertiäre Amine sind Trialkylamine, deren Alkylreste eine Kettenlänge von C₁-C₁₈ aufweisen.
Die Alkylgruppen der tertiären Amine können dabei auch zu einem Ring geschlossen sein, so dass cyclische tertiäre Amine vorliegen. Ein bevorzugtes cyclisches tertiäres Amin ist Piperidin. Im Hinblick auf das erfindungsgemäße Verfahren ist ein substituiertes Pyridin einem tertiären Amin gleichwertig, da ein Pyridin gleiche, oder zumindest ähnliche Reaktionen eingeht, die typischerweise auch ein tertiäres Amin eingeht. So können auch benzoanellierte Ringsystem des Pyridins eingesetzt werden. Bei Pyridinen ist es bevorzugt, wenn diese zur Gewährleistung ihrer Löslichkeit mindestens durch einen Alkyl-, Alkoxy, oder Halogenrest substituiert sind. Als Substituenten des Pyridins sind Alkylreste und Alkoxyreste mit einer Kettenlänge von C₁-C₁₈ besonders bevorzugt.

Neben Pyridin sind prinzipiell auch andere Heteroaromaten zur Durchführung des erfindungsgemäßen Verfahrens geeignet. Dabei muss die Basizität dieser Heteroaromaten gleich oder höher sein als die Basizität des Anilins (Q).

Die genannten Stickstoffbasen ohne NH-Gruppe können alternativ oder in Kombination miteinander eingesetzt werden. Ein Gemisch kann neben einem tertiären Amin, ein substituiertes Pyridin und/oder ein benzoanelliertes Ringsystem des Pyridins umfassen. Alternativ kann das Basengemisch ein substituiertes Pyridin und/oder ein benzoanelliertes Ringsystem des Pyridins umfassen.

Die Pyridinsubstituenten sind bevorzugt ausgewählt aus der Gruppe bestehend aus Alkyl-, Alkoxy oder Halogenresten, wobei bei Alkylresten und Alkoxyresten eine Kettenlänge von C₁-C₁₈ besonders bevorzugt ist.

Besonders bevorzugte tertiäre Amine sind Trialkylamine, deren Alkylreste eine Kettenlänge von C₁-C₁₈ aufweisen, wobei mindestens einer der Reste eine Kettenlänge von mindestens C₄-C₁₈ aufweist. Die genannte Mindestkettenlänge von sechs C-Atomen bei mindestens einem der drei Reste des Trialkylamins stellt sicher, dass das tertiäre Amin ausreichend lipophil ist, um die Löslichkeit des tertiären Amins im dem gewählten organischen Lösungsmittel zu gewährleisten. Falls die beiden verbleibenden Reste des Reste des Trialkylamins ebenfalls ein Mindestkettenlänge von sechs C-Atomen aufweisen, ist die Lipophilie des Amins entsprechend höher. Dabei können auch entsprechende Kombinationen der Kettenlängen aller Alkylreste auf eine Gesamtkettenlänge von mindestens C₄-C₁₈ ausreichen, um die Löslichkeit des HCl-Salzes zu gewährleisten. Die Alkylreste des Trialkylamins können dabei jeweils unsubstituiert oder substituiert sein.

Ein für das vorliegende Verfahren ganz besonders bevorzugtes Trialkylamin ist Tributylamin.

Ein ganz besonders bevorzugtes Pyridin ist 2-Methyl-5-Ethyl-Pyridin.

Ein ganz besonders bevorzugtes benzoanelliertes Ringsystem des Pyridins, ist substituiertes oder unsubstituiertes Chinolin, wobei unsubstituiertes Chinolin am meisten bevorzugt ist.

Es ist auch denkbar, dass das Verfahren in einem Gemisch aus Stickstoff-Basen durchgeführt wird, wobei das Gemisch entweder aus
- einem Tributylamin und 2-Methyl-5-Ethyl-Pyridin,
- einem Tributylamin und einem unsubstituierten Chinolin, oder aus
- einem Gemisch aus 2-Methyl-5-Ethyl-Pyridin und einem unsubstituierten Chinolin
besteht.

Alternativ ist auch ein aus drei der genannten Stickstoff-Basen bestehendes Gemisch einsetzbar. Das am meisten bevorzugte Basengemisch mit drei Stickstoff-Basen umfasst Tributylamin, ein unsubstituiertes Chinolin und 2-Methyl-5-Ethyl-Pyridin.

Das erfindungsgemäße Verfahren kann mit verschiedenen Lösungsmitteln durchgeführt werden, vorausgesetzt dass das HCl-Salz der Stickstoff-Base bei der gewählten Reaktionstemperatur in diesem Lösungsmittel gelöst ist.
Die Erfüllung der vorgenannten Voraussetzung ist wesentlich für die Durchführung des Verfahrens. Außerdem muss das Lösungsmittel mit dem Chlorierungsmittel kompatibel sein.

Diese Anforderungen erfüllen unpolare organische Lösungsmittel, wie
- Chloralkane (beispielsweise Dichlormethan und Dichlorethan),
- Aromaten (beispielsweise Benzol, Toluol, Xylol),
- Haloaromaten (beispielsweise Chlorbenzol, Dichlorbenzol),
- substituierte Aromaten (beispielsweise Benzotrifluorid, Chlorbenzotrifluorid, Chlortoluol, Chlorxylol) allein, oder
- eine Mischung, die eines oder mehrere der vorgenannten unpolaren anorganischen Lösungsmittel umfasst.

Neben unpolaren organischen Lösungsmitteln sind, wie in WO 2010/127786 A1 beschrieben, auch polare organische Lösungsmittel, vorzugsweise Ester-Lösungsmittel, wie zum Beispiel (C₁-C₆) Alkylacetat (beispielsweise Methylacetat, Ethylacetat, n-Propylacetat, iso-Propylacetat, 2-Methyl-Prop-1-ylacetat, n-Butylacetat, But-2-ylacetat, Pentylacetate, Hexylacetate und Cyclalkylacetate, (C₁-C₆) Alkyl und Cycloalkylpropionate, (C₁-C₆) Alkyl und Cycloalkyl -n-butyrate, -iso-butyrate, -pentanoate und -hexanoate und -cyclopentanoate und -cyclohexanoate) oder eine Mischung, die eines oder mehrere der vorgenannten unpolaren organischen Lösungsmittel umfasst, zur Durchführung der erfindungsgemäßen Reaktion geeignet.

Als Chlorierungsmittel kommen alle in dem organischen Lösungsmittel löslichen, dem Fachmann hierfür bekannten Chlorierungsmittel in Frage.

Bevorzugte Chlorierungsmittel sind tert-Butylhypochlorit und Sulfurylchlorid, die vorteilhafterweise beide flüssig sind und außerdem in einem der genannten Lösungsmittel gut löslich sind. Die beiden genannten Chlorierungsmittel können auch gemeinsam, d.h. im Gemisch miteinander, eingesetzt werden. Besonders bevorzugt ist das Chlorierungsmittel Sulfurylchlorid (SO₂Cl₂).

In einer bevorzugten Ausführungsform des Verfahrens ist vorgesehen, dass in einer ersten Mischkammer vor der Vereinigung der beiden Edukte und vor Zugabe des Chlorierungsmittels mindestens eines der beiden Edukte
- in einem organischen Lösungsmittel gelöst wird, und
- mit mindestens einer Stickstoff-Base, die keine NH-Gruppe aufweist und in dem gewählten organischen Lösungsmittel bei der jeweiligen Reaktionstemperatur gelöst vorliegt, gemischt wird.

Ein wesentlicher Aspekt des erfindungsgemäßen Verfahrens betrifft dessen Durchführung als eine kontinuierlich ablaufende Reaktion in einem Flussreaktor. Die Möglichkeit der mehrfachen Zugabe, d.h. der wiederholten Zugabe, einer Stickstoff-Base ohne NH-Gruppe, bzw. eines Gemisch derselben, in verschiedenen Reaktionsstadien ist ein wesentlicher Parameter bei der Verfahrensführung. Bei Durchführung der kontinuierlichen Reaktion in einem Flussreaktor werden die Reaktionsstadien definiert durch die verschiedenen zum Flussreaktor gehörenden Vorlagekammern, Mischkammern und Verweilstrecken, die dem Reaktionsablauf entsprechend aufeinanderfolgend angeordnet sind.

Grundsätzlich wird die Base den Edukten bereits in der, bzw. den Vorlagekammern, zugegeben.

In einer bevorzugten Ausführungsform kann dem Reaktionsgemisch außerdem nach Vereinigung der beiden in einem organischen Lösungsmittel gelösten Edukte und nach Zugabe des Chlorierungsmittels, jedoch vor Beschickung der zum Flussreaktor gehörenden Verweilstrecke mit dem Reaktionsgemisch, die Stickstoff-Base erneut zugegeben werden.

Durch die mehrfache Zugabe der Stickstoff-Base wird sichergestellt, dass die Viskosität des Reaktionsgemischs den Betriebsanforderungen im jeweiligen Abschnitt des Flussreaktors, d.h. in den Vorlagekammern, den Mischkammern und den Verweilstrecken, entspricht.

Die Löslichkeit des Reaktionsgemischs in den Verweilstrecken eines Flussreaktors ist besonders wichtig für die Anwendbarkeit des Flussreaktors. Das gilt insbesondere für die Gewährleistung der Betriebsanforderungen der ersten zu einem Flussreaktor gehörenden Verweilstrecke.

Dementsprechend liegt es im Rahmen des erfindungsgemäßen Verfahrens, dass dem Reaktionsgemisch, nachdem dieses die erste zum Flussreaktor gehörende Verweilstrecke passiert hat, und bevor die zweite zum Flussreaktor gehörende Verweilstrecke mit dem Reaktionsgemisch beschickt wird, eine Stickstoff-Base, die keine NH-Gruppe aufweist und deren HCl-Salz in dem gewählten organischen Lösungsmittel bei der jeweiligen Reaktionstemperatur gelöst vorliegt, erneut zugegeben wird.

Die erneute Zugabe bewirkt, dass die Viskosität des Reaktionsgemischs, den Betriebsanforderungen eines Flussreaktors, insbesondere den Betriebsanforderungen einer zweiten zu einem Flussreaktor gehörenden Verweilstrecke, entspricht.

Es liegt im Rahmen der Erfindung, dass dem jeweiligen Reaktionsgemisch nach dem Verlassen einer Verweilstrecke, d.h. vor dem Beschicken der nachfolgenden Verweilstrecke, jeweils erneut eine Stickstoff-Base, die keine NH-Gruppe aufweist und deren HCl-Salz in dem gewählten organischen Lösungsmittel bei der jeweiligen Reaktionstemperatur gelöst vorliegt, zugegeben wird.

Die Durchführung der erneuten, d.h. der wiederholten, Zugabe der Stickstoff-Base wird durch das Synthesebeispiel 1 belegt und entspricht der nachfolgend erläuterten ganz besonders bevorzugten Ausführungsform des erfindungsgemäßen Verfahrens.

In einer ganz besonders bevorzugten Ausführungsform des Verfahrens ist vorgesehen, dass in einer ersten Vorlagekammer (VO1) des Flussreaktors als Vorlage-Gemisch-1 (VG1), mindestens enthaltend:
- ein Anilin (Q): 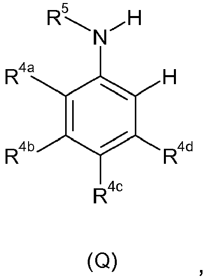 worin die Reste R^{4a} bis R^{4d} und R⁵ wie in Formel (4) definiert sind,
- ein organisches Lösungsmittel, in dem das Anilin (Q) gelöst ist, und
- mindestens eine Stickstoff-Base, die keine NH-Gruppe aufweist, vorgelegt wird, und
in einer zweiten Vorlagekammer (VO2) des Flussreaktors ein Vorlage-Gemisch-2 (VG2), mindestens enthaltend:
- ein flüssiges Chlorierungsmittel und
- ein organisches Lösungsmittel, das dem in der ersten Vorlagekammer befindlichen Lösungsmittel entspricht,
vorgelegt wird, und
- nachfolgend das Vorlage-Gemisch-1 (VG1) und das Vorlage-Gemisch-2 (VG2) in einer ersten Mischkammer (M1) bei einer Reaktionstemperatur im Bereich zwischen - 65°C und 0°C gemischt werden, und
- nachfolgend in einer zweiten Mischkammer (M2) das Reaktionsgemisch aus der ersten Mischkammer (M1) mit einem in einer dritten Vorlagekammer (VO3) eines Flussreaktors vorgelegten Vorlage-Gemisch-3 (VG3), mindestens enthaltend:
   - einen Thioether (W) 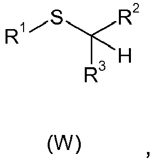 worin die Reste R¹, R² und R³ wie in Formel (4) definiert sind,
   - ein organisches Lösungsmittel, das dem in der ersten Vorlagekammer vorgelegten Lösungsmittel entspricht, und
   - eine Stickstoff-Base, die keine NH-Gruppe aufweist und der in der ersten Vorlagekammer vorgelegten Stickstoffbase entspricht.
   vereinigt wird, und das so erhaltene Gemisch
   - nachfolgend in einem ersten Verweilelement-1 (VW-1) des Flussreaktors umgesetzt werden, und anschließend
   - das in dem Verweilelement-1 (VW-1) umgesetzte Gemisch in einer dritten Mischkammer (M3) des Flussreaktors erneut mit einer Stickstoff-Base, die keine NH-Gruppe aufweist, und der in der ersten Vorlagekammer vorgelegten Stickstoffbase entspricht, gemischt wird, und das so erhaltene Gemisch
   - nachfolgend erneut in einem zweiten Verweilelement-2 (VW-2) des Flussreaktors zu einer Verbindung der Formel (4) umgesetzt wird.

Die vorgenannten Mischungen aus einem Anilin (Q) und einer Stickstoff-Base ohne NH-Gruppe, bzw. aus einem Thioether (W) und einer Stickstoff-Base ohne NH-Gruppe, werden vorzugsweise vorher mit einem Lösungsmittel oder einem Lösungsmittelgemisch verdünnt, wobei die Mischung abgekühlt werden kann, indem bereits die Zuleitungen zu den Mischkammern (M) vorgekühlt werden.

Ebenso kann allein die Stickstoff-Base (Menge Z") mit einem Lösungsmittel oder alternativ mit einem Lösungsmittelgemisch vorverdünnt werden und zusätzlich vorgekühlt werden, indem die Zuleitungen zu den Mischkammern (M) vorgekühlt werden.

Auch das Chlorierungsmittel kann mit einem Lösungsmittel oder einem Lösungsmittelgemisch vorverdünnt werden und kann vorzugsweise vorgekühlt werden, indem die Zuleitungen zu den Mischkammern vorgekühlt werden.

Die Einsatzmengen der beiden Edukte, d.h. des Anilins der Formel (Q) und des Thioethers der Formel (W), sowie die Einsatzmengen des Chlorierungsmittels und der Stickstoff-Base sind jeweils über einen weiten Bereich variabel.

Bevorzugt sind die nachfolgend genannten Einsatzmengen: Bis zu 1 Äquivalent, bevorzugt 0,5 bis 1,0 Äquivalente, besonders bevorzugt 0,7 bis 1,0 Äquivalente, insbesondere bevorzugt 0,8 bis 0,95 Äquivalente des Chlorierungsmittels werden mit einer Mischung enthaltend 1 Äquivalent des Anilins (Q) und bis zu 1 Äquivalent, bevorzugt 0,1 bis 0,9 Äquivalente, besonders bevorzugt 0,2 bis 0,5 Äquivalente, insbesondere bevorzugt 0,25 bis 0,35 Äquivalente der Stickstoff-Base (Menge Z) in einer Mischkammer eines Flussreaktors gemischt.

Das so erhaltene Gemisch wird direkt mit einer Mischung von einem Äquivalent des Thioethers (W) und bis zu 1 Äquivalent, bevorzugt 0,1 bis 0,9 Äquivalente, besonders bevorzugt 0,5 bis 0,8 Äquivalente, insbesondere bevorzugt 0,65 bis 0,75, Äquivalenten der Stickstoff-Base (Menge Z') jeweils in der Mischkammer eines Flussreaktors gemischt, wobei die gesamt Menge an Amin (Z+Z') größer oder gleich den Äquivalenten des Chlorierungsmittels ist. Nach Durchlaufen einer Verweilstrecke wird die Mischung mit bis zu 2 Äquivalenten, bevorzugt 0,5 bis 1,8 Äquivalenten, besonders bevorzugt 1,0 bis 1,6 Äquivalenten, der Stickstoff-Base (Menge Z") gemischt.

Nachfolgend werden die im Zusammenhang mit der vorliegenden Reaktion eingesetzten Edukte sowie die in dieser Beschreibung verwendeten chemischen Begriffen näher erläutert.

Das im erfindungsgemäßen Verfahren eingesetzte Anilin (Q) hat die folgende Struktur: 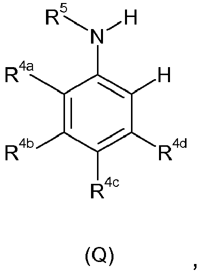 worin
R^{4a} bis R^{4d} unabhängig voneinander ausgewählt sind aus der Gruppe bestehend aus Wasserstoff, Fluor, Chlor, Brom, Iod, CN, NO₂ sowie aus
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
CO-X, wobei X für OR^{1"}, SR¹ oder NR^{2'}R^{2"} steht, worin R^{1"} wie R¹ definiert ist und R^{1"} gleich oder anders ist als R¹, und worin R^{2'} und R^{2"} unabhängig voneinander jeweils für H, (C₁-C₆) Alkyl, substituiertes (C₁-C₆) Alkyl, Aryl oder substituiertes Aryl stehen oder R^{2'} und R^{2"} einen Ring bilden,
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R⁵ für H, (C₁-C₆) Alkyl, substituiertes (C₁-C₆) Alkyl, Aryl oder substituiertes Aryl steht.

Soweit in dieser Beschreibung auf ein "Anilin" Bezug genommen wird, ist eine der oben genannten Verbindungen der Formel (Q) gemeint. Voraussetzung zur Durchführung des Verfahrens ist, dass das eingesetzte Anilin in ortho-Position zur Aminogruppe ein Wasserstoffatom aufweist, d.h. das eingesetzte Anilin muss in der ortho-Position unsubstituiert sein. Im Übrigen kann der aromatische Ring des Anilins neben der Aminogruppe bis zu vier weitere Substituenten R^{4a} bis R^{4d} aufweisen. Besonders bevorzugt ist eine zweifache Substitution des Anilins (Q), d.h. zwei der Reste R^{4a} bis R^{4d} sind ungleich H.

Besonders bevorzugt sind Aniline der Formel (Q), in welchen die Reste R^{4a} bis R^{4d} unabhängig voneinander jeweils für H, F, Cl, Br, I, CF₃, CN, NO₂ oder CO-X stehen, wobei X für OR^{1"}, SR^{1"} oder NR^{2'}R^{2"} steht, worin R^{1"} wie R¹ definiert ist und R^{1"} gleich oder anders ist als R¹, und worinR^{2'} und R^{2"} wie R² definiert sind, wobei R^{2'} und R^{2"} jeweils gleich oder anders sind als R², oder R^{2'} und R^{2"} einen Ring bilden.

Ganz besonders bevorzugt ist die nur einfache Substitution des Anilins, d.h. einer der Reste R^{4a} bis R^{4d} ist ungleich H, so dass die Anilin-Edukte am aromatischen Ring des Anilins neben der Aminogruppe einen weiteren Substituenten aufweisen.

Besonders bevorzugt als Rest R^{4a} bis R^{4d} ist Fluor. Ganz besonders bevorzugt ist, wenn R^{4a}, d.h. der Rest in ortho-Stellung zur Amino-Gruppe ein Fluor ist, wobei der aromatische Ring des Anilins im Übrigen nicht substituiert ist.

Soweit in dieser Beschreibung auf einen "Thioether" Bezug genommen wird, ist eine der oben genannten Verbindungen der Formel (W) gemeint. Der im erfindungsgemäßen Verfahren eingesetzte Thioether (W) hat die folgende Struktur: 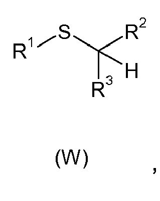 worin
R¹ für (C₁-C₆) Alkyl, substituiertes (C₁-C₆) Alkyl, Aryl oder substituiertes Aryl steht,
R² für einen elektronenziehenden oder aktivierenden Rest, ausgewählt aus der Gruppe bestehend aus
   - CN,
   - NO₂,
   - CO-R^{1'}, wobei R^{1'} wie R¹ definiert ist und R^{1'} gleich oder anders ist als R¹,
   - CO-X, wobei X für OR^{1"}, SR^{1"} oder NR^{2'}R^{2"} steht, worin R^{1"} wie R¹ definiert ist und R^{1"} gleich oder anders ist als R¹, und worin R^{2'} und R^{2"} unabhängig voneinander jeweils für H, (C₁-C₆) Alkyl, substituiertes (C₁-C₆) Alkyl, Aryl oder substituiertes Aryl stehen oder R^{2'} und R^{2"} alternativ einen Ring bilden,
   - SO(n)-R1^{'"}, worin R^{1"'} wie R¹ definiert ist, wobei R^{1"'} jeweils gleich oder anders ist als R¹ und wobei n 0, 1, oder 2 ist,
   - Aryl, und
   - Heteroaryl, und
R³ für H, (C₁-C₆) Alkyl, substituiertes (C₁-C₆) Alkyl, Aryl oder substituiertes Aryl steht.

Die erfindungsgemäß einsetzbaren Verbindungen der Formeln (Q) und (W) sind nach den dem Fachmann bekannten Verfahren herstellbar.

Im Zusammenhang mit den in dieser Beschreibung verwendeten chemischen Begriffen gelten die für den Fachmann üblichen Definitionen, sofern nichts anderes spezifisch definiert ist.

Die Reste Alkyl, Alkoxy, Haloalkyl, Haloalkoxy, Alkylamino und Alkylthio sowie die entsprechenden ungesättigten und/oder substituierten Reste im Kohlenstoffgerüst können jeweils geradkettig oder verzweigt sein. Wenn nicht speziell angegeben, sind bei diesen Resten die niederen Kohlenstoffgerüste, z.B. mit 1 bis 6 C-Atomen, bzw. bei ungesättigten Gruppen mit 2 bis 6 C Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl.

Cycloalkyl bedeutet ein carbocyclisches, gesättigtes Ringsystem mit vorzugsweise 3 bis 8 C-Atomen, z.B. Cyclopropyl, Cyclobutyl, Cyclopentyl oder Cyclohexyl.

Halogen bedeutet Fluor, Chlor, Brom oder Iod. Haloalkyl bedeutet durch Halogen, vorzugsweise durch Fluor, Chlor und/oder Brom, insbesondere durch Fluor und/oder Chlor, teilweise oder vollständig substituiertes Alkyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Ein substituiertes Alkyl steht für ein (C₁-C₆) Alkyl, das substituiert ist durch einen oder mehrere Reste ausgewählt aus der Gruppe bestehend aus (C₁-C₆) Alkyl, (C₁-C₆) Alkoxy, Aryl, Heteroaryl, und Halogen.

Ein substituiertes Aryl steht für ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl, das substituiert ist mit einem oder mehreren Substituenten, ausgewählt aus der Gruppe bestehend aus (C₁-C₆) Alkyl, (C₁-C₆) Alkoxy, Aryl, Heteroaryl und Halogen.

Ein heterocyclischer Rest oder Ring (Heterocyclyl) kann gesättigt, ungesättigt oder heteroaromatisch sein; er enthält vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono, bi oder polycyclisches aromatisches System, in dem mindestens 1 Ring ein oder mehrere Heteroatome enthält, beispielsweise Pyridyl, Pyrimidinyl, Pyridazinyl, Pyrazinyl, Triazinyl, Thienyl, Thiazolyl, Thiadiazolyl, Oxazolyl, Isoxazolyl, Furyl, Pyrrolyl, Pyrazolyl und Imidazolyl, oder ist ein partiell oder vollständig hydrierter Rest wie Oxiranyl, Pyrrolidyl, Piperidyl, Piperazinyl, Dioxolanyl, Oxazolinyl, Isoxazolinyl, Oxazolidinyl, Isoxazolidinyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie ein substituierter Alkyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, Sulfamoyl, Mono und Dialkylaminosulfonyl, substituiertes Amino, wie Acylamino, Mono und Dialkylamino, und Alkylsulfinyl, Haloalkylsulfinyl, Alkylsulfonyl, Haloalkylsulfonyl und, im Falle cyclischer Reste, auch Alkyl und Haloalkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Phenyl, Phenoxy etc. eingeschlossen. Bei Resten mit C Atomen sind solche mit 1 bis 4 C Atomen, insbesondere 1 oder 2 C Atomen, bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl und Fluor.

Von den Formeln (4) sind, soweit zutreffend, auch alle Stereoisomeren umfasst. Solche Verbindungen enthalten ein oder mehrere asymmetrische C-Atome, die in den allgemeinen Formeln nicht gesondert angegeben sind. Die durch ihre spezifische Raumform definierten möglichen Stereoisomeren, wie Enantiomere, Diastereomere, können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Die erfindungsgemäß einzusetzenden Aniline der Formel (Q) und Thioether der Formel (W) können nach den dem Fachmann bekannten Verfahren hergestellt werden.

Ein weiterer Aspekt der Erfindung betrifft ein mehrstufiges Verfahren zur Herstellung von Verbindungen der Formel (1-1), bzw. von Verbindungen der Formel (4-1), welche jeweils herbizide Wirkung aufweisen, ausgehend von Verbindungen der Formel (4), bzw. Verbindungen der Formel (4').
Bei dem mehrstufigen Verfahren werden zunächst die Verbindungen der Formel (4) in Gegenwart eines Säure-Katalysators zu einem Oxindol der Formel (7-1) umgesetzt. Anschließend werden die Oxindole der Formel (7-1) in den Schritten gemäß Schema 5 zu Verbindungen der Formel (1-1) und N-Alkyl-N-[2-(1,3,5-triazin-2-ylcarbonyl)phenyl] alkansulfonamiden der Formel (4-1) umgesetzt.

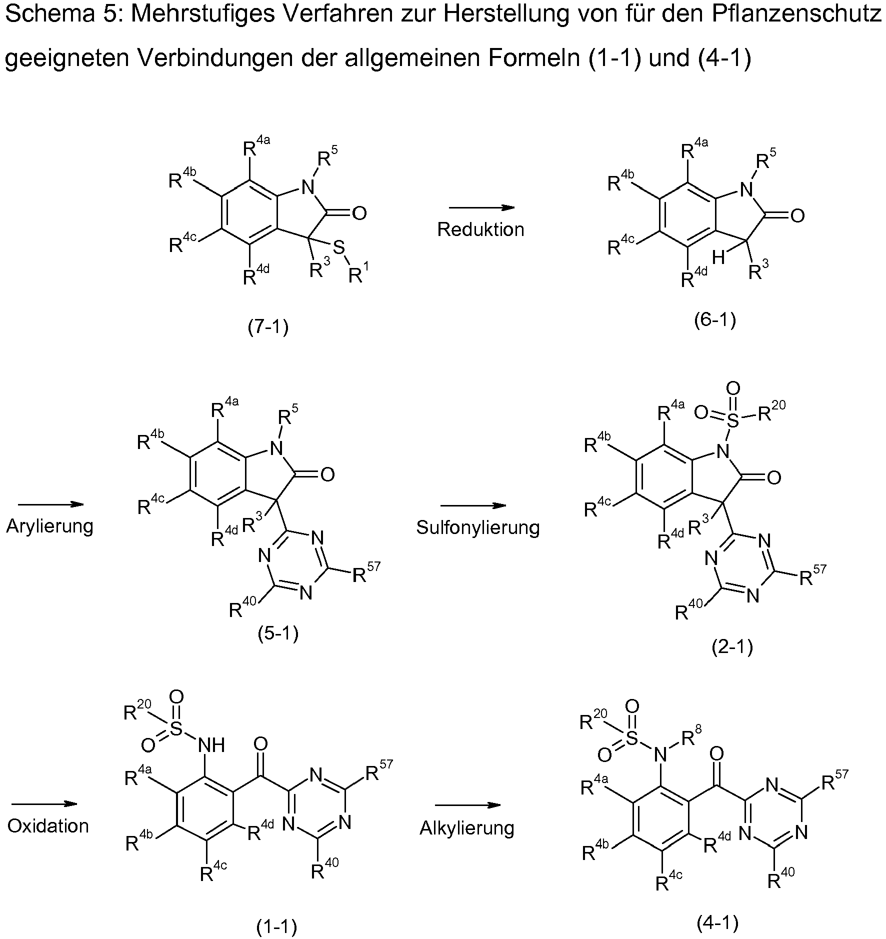

Das mehrstufige Verfahren zeichnet sich gegenüber den vorbekannten Verfahren zur Herstellung von N-Alkyl-N-[2-(1,3,5-triazin-2-ylcarbonyl)phenyl]-alkansulfonamiden (4-1) sowie von 2-(Triazinylcarbonyl)sulfon- aniliden (1-1) dadurch aus, dass als Edukte, bzw. als Intermediate, die mittels einer kontinuierlichen Reaktionsführung in einem Flussreaktor gewonnenen ortho-substituierten Aniline der Formel (4), bzw. Oxindolverbindungen der Formel (7-1), eingesetzt werden. Ein Aspekt der Erfindung umfasst somit auch die Verwendung der in einem Flussreaktor gewonnenen ortho-substituierten Aniline der Formel (4) zur Herstellung von N-Alkyl-N-[2-(1,3,5-triazin-2-ylcarbonyl)phenyl]alkansulfonamiden der Formel (4-1).

Der Einsatz eines Flussreaktors hat den Vorteil, dass das Verfahren zur Herstellung von Verbindungen der Formel (4-1) im Vergleich zu den vorbekannten Verfahren im technischen Maßstab noch effizienter durchgeführt werden kann und zugleich hohe Ausbeuten erhalten werden können.

Die Durchführung des mehrstufigen Verfahrens (Gesamtverfahren) wird nachfolgend erläutert: Zunächst wird eine gemäß Anspruch 1 unter dem Einsatz eines Flussreaktors in einem kontinuierlichen Verfahren gewonnene Verbindung der Formel (4) in Gegenwart eines Säure-Katalysators zu einem 3-(Alkylsulfanyl)-1,3-dihydro-2H-indol-2-on der Formel (7-1) umgesetzt. Die Herstellung von Oxindolen der Formel (7-1) ausgehend von Verbindungen der Formel (4), bzw. hier beispielhaft der Formel (4'), in welchen R² bevorzugt für CO₂R, worin R wiederum besonders bevorzugt für Methyl oder Ethyl steht (siehe nachfolgendes Schema), ist dadurch gekennzeichnet, dass die gemäß Anspruch1 hergestellten Verbindungen der Formel (4) nach Extraktion des tertiären Amins entweder
- direkt in der Reaktionslösung, oder
- nach dem Aufkonzentrieren der Reaktionslösung mit einer alkoholischen HCl-Lösung versetzt werden und bei einer Temperatur zwischen 0 bis 40°C nachgerührt werden.

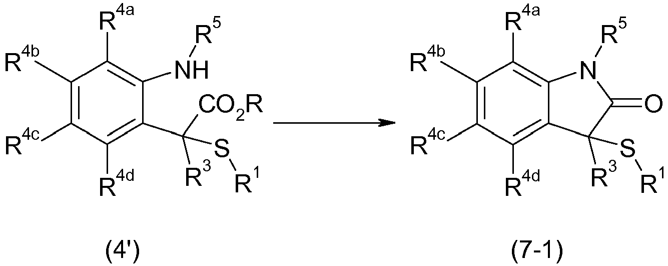

Alternativ erfolgt die Herstellung von Oxindolen der Formel (7-1) ausgehend von Verbindungen der Formel (4), in welchen R²für CO-X steht, wobei X SR^{1"} oder NR^{2'}R^{2"} steht, worin R^{1"} wie R¹ definiert ist und R^{1"} gleich oder anders ist als R¹, und worin R^{2'} und R^{2"} unabhängig voneinander jeweils für H, (C₁-C₆) Alkyl, substituiertes (C₁-C₆) Alkyl, Aryl oder substituiertes Aryl stehen oder R^{2'} und R^{2"} einen Ring bilden.

Anschließend wird das gemäß dem vorgenannten Vorgehen aus einer Verbindung der Formel (4), bzw. (4'), erhaltene 3-(Alkylsulfanyl)-1,3-dihydro-2H-indol-2-on der Formel (7-1) 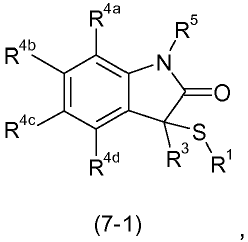 worin
R¹ für ein unsubstituiertes (C₁-C₆) Alkyl, ein substituiertes (C₁-C₆) Alkyl, ein unsubstituiertes Aryl oder ein substituiertes Aryl steht,
R³ für Wasserstoff steht,
R^{4a} bis R^{4d} wie für Formel (4) definiert sind,
R⁵ für Wasserstoff steht,
durch
- Reduktion zu einem 1,3-Dihydro-2H-indol-2-on (6-1) 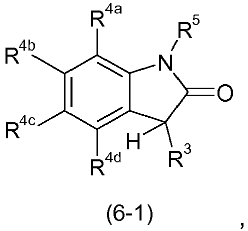 worin R^{4a} bis R^{4d}, R³ und R⁷ wie für Formel (7-1) definiert sind, umgewandelt.

Bevorzugt ist die Umsetzung von Verbindungen der Formel (7-1), in welchen R⁵ für ein unsubstituiertes oder substituiertes (C₁-C₄)-Alkyl, ein (C₃-C₇)-Cycloalkyl, ein Benzyl oder ein CH₂-C(O)O-(C₁-C₆)-Alkyl steht.

Die mittels Reduktion erhaltenen Verbindungen der Formel (6-1) werden gemäß den in Schema 5 zusammengefassten Reaktionsschritten, d.h. die Schritte der Arylierung, Sulfonylierung, Oxidation sowie der Alkylierung, zu einem Herbizid der Formel (4-1) umgesetzt.

Zur Herstellung von N-Alkyl-N-[2-(1,3,5-triazin-2-ylcarbonyl)phenyl]alkansulfonamiden der Formel (4-1) 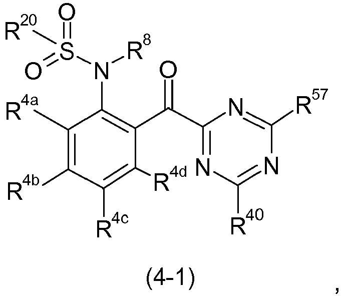 worin
R^{4a} bis R^{4d} wie für Verbindungen der Formel (4), bzw. der Formel (Q) definiert sind, und
R⁸ für
   (C₁-C₆)-Alkyl, wobei der Alkylrest unsubstituiert ist oder ganz oder teilweise mit Fluor substituiert ist,
   (C₁-C₆)-Cycloalkyl, (C₁-C₆)-Alkenyl oder (C₁-C₆)-Alkoxyalkyl, wobei jeder der genannten Reste unsubstituiert ist oder ganz oder teilweise mit Fluor substituiert ist,
   steht, und
R²⁰ für
   (C₁-C₆)-Alkyl, wobei der Alkylrest unsubstituiert ist oder ganz oder teilweise mit Fluor substituiert ist, oder
   (C₃-C₇)-Cycloalkyl, wobei der Cycloalkylrest unsubstituiert ist oder ganz oder teilweise mit Fluor substituiert ist, stehen, und
R⁴⁰ und R⁵⁷ unabhängig voneinander jeweils für
   Wasserstoff,
   (C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   (C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
   stehen,
   ausgehend von einem 1,3-Dihydro-2H-indol-2-on der Formel (6-1) 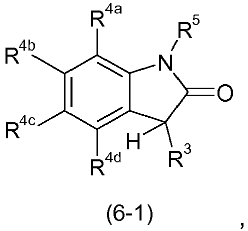 worin
R^{4a} bis R^{4d} wie für Formel (4) definiert sind,
R³ für Wasserstoff steht, und
R⁵ für Wasserstoff steht, werden in einem
ersten Schritt durch
   - Arylierung zu einem triazinylsubstituierten Oxindol der Formel (5-1) 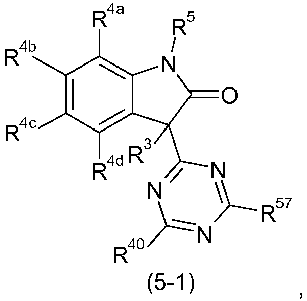 worin R^{4a} bis R^{4d} und R⁴⁰ und R⁵⁷ wie für die Formel (4-1) und R³ und R⁷ wie für die Formel (6-1) definiert sind,
   umgesetzt, und die Arylierungsprodukte der Formel (5-1) in einem
   zweiten Schritt durch
   - Sulfonylierung zu N-sulfonylsubstituierten 3-Triazinyloxindolen der Formel (2-1) 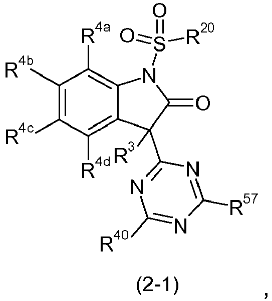 worin
      R^{4a} bis R^{4d}, R²⁰ sowie R⁴⁰ und R⁵⁷wie in Formel (4-1) und R³ wie für die Formel (6-1)definiert sind,
      umgesetzt, und die Sulfonylierungsprodukte der Formel (2-1) in einem dritten Schritt durch
   - oxidative Ringöffnung zu einem 2-(Triazinylcarbonyl)sulfonanilid der Formel (1-1)
   - X- R⁸, worin X für Chlor, Brom, Iod oder OSO₂R⁹ steht, wobei R⁸ wie oben für Formel (4-1) definiert ist und R⁹ wie oben R¹ definiert ist, oder
   - (R⁸)₂SO₄, worin R⁸ wie oben für Formel (4-1) definiert ist, eingesetzt wird.

Somit umfasst das mehrstufige Verfahren zur Herstellung von N-Alkyl-N-[2-(1,3,5-triazin-2-ylcarbonyl) phenyl]alkansulfonamiden der Formel (4-1), neben dem gemäß Anspruch 1 der vorliegenden Erfindung in einem Flussreaktor durchgeführten Verfahren zur Herstellung von Oxindolen der Formel (7-1) fünf weitere Teilschritte, welche jeweils Gegenstand früherer Anmeldungen sind. Diese Teilschritte und deren Ausführung werden nachfolgend kurz erläutert:
- Reduktion von substituierten oder unsubstituierten 3-(Alkylsulfanyl)-1,3-dihydro-2H-indol-2-onen (7-1) zu substituierten oder unsubstituierten 1,3-Dihydro-2H-indol-2-onen (6-1). Dieses Verfahren ist im technischen Maßstab möglich und ist in der Patentanmeldung mit der Anmeldenummer EP 10162381.7 beschrieben.

Die Reduktion ist dadurch gekennzeichnet, dass
a) eine Verbindung der Formel (7-1) in einem polaren Lösungsmittel gelöst oder suspendiert wird,
b) der Lösung oder der Suspension ein schwefelhaltiges Salz zugesetzt wird,
   und
c) das Reaktionsgemisch bei einer Temperatur, die maximal der Siedetemparatur des polaren Lösungsmittels entspricht, unter Rückfluss erwärmt wird.

Als schwefelhaltige Salze sind zur Durchführung der Reduktion besonders bevorzugt Natriumsalze, ausgewählt aus der Gruppe bestehend aus Natriumbisulfit, Natriumsulfit, Natriumthionit, Natriumdithionit und Natriumthiosulfat.
- Arylierung von substituierten oder unsubstituierten 1,3-Dihydro-2H-indol-2-onen (6-1) zu triazinylsubstituierten Oxindolen (5-1). Dieses Verfahren ist im technischen Maßstab möglich und ist in der Patentanmeldung mit der Anmeldenummer EP 10196205.8 beschrieben.

Die Arylierung ist dadurch gekennzeichnet, dass sie in Gegenwart
- eines Carbonats, oder
- eines Hydroxids, oder
- eines Phosphats, oder
- in einer mindestens zwei der vorgenannten Basen umfassenden Mischung durchgeführt wird.

Bevorzugt werden bei der Arylierung als Basen Kaliumcarbonat, Natriumcarbonat, Natriumhydroxid, Kaliumhydroxid oder eine mindestens zweikomponentige Mischung bestehend aus mindestens einem der beiden Carbonate: Kaliumcarbonat und Natriumcarbonat sowie aus mindestens einem der beiden Hydroxide: Kaliumhydroxid oder Natriumhydroxid, eingesetzt.
- Sulfonylierung von triazinylsubstituierten Oxindolen (5-1) zu N-sulfonylsubstituierten 3-Triazinyloxindolen (2-1). Dieses Verfahren ist im technischen Maßstab möglich und ist in der Patentanmeldung mit der Anmeldenummer EP 11159875.1 beschrieben.

Die Sulfonylierung ist dadurch gekennzeichnet, dass sie in Gegenwart
- einer in 1-Position substituierten Imidazol-Base, oder
- eines Basen-Gemischs, das mindestens eine in 1-Position substituierte Imidazol-Base enthält, erfolgt.

Besonders bevorzugte Imidazol-Basen für die Durchführung der Sulfonylierung sind 1-Methyl-1H-Imidazol, 1-Butyl-1H-Imidazol oder 1-Benzyl-1H-Imidazol, die einzeln oder im Gemisch eingesetzt werden können, wobei die Verwendung von 1-Methyl-1H-Imidazol ganz besonders bevorzugt ist.
- Oxidative Ringöffnung von N-sulfonylsubstituierten 3-Triazinyloxindolen (2-1) zu 2-(Triazinylcarbonyl)sulfonaniliden (1-1). Dieses Verfahren ist im technischen Maßstab möglich und ist Gegenstand der Patentanmeldung mit dem Aktenzeichen PCT/EP2011/073287.
- Alkylierung von 2-(Triazinylcarbonyl)sulfonaniliden (1-1) zu N-Alkyl-N-[2-(1,3,5-triazin-2-ylcarbonyl)phenyl]alkansulfonamiden (4-1). Dieses Verfahren ist in der Patentanmeldung WO 2006/008159 A1 beschrieben.

Die Alkylierung kann mit gängigen Alkylierungsmitteln erfolgen. Im Falle einer Methylierung wird bevorzugt Dimethylsulfat eingesetzt.

Die herbizide Wirkung (siehe WO 2007/031208 A2) und fungizide Wirkung (siehe WO 2006/008159 A1) von N-Alkyl-N-[2-(1,3,5-triazin-2-ylcarbonyl)phenyl]alkansulfonamiden der Formel (4-1) ist seit längerem bekannt.

Somit wird durch die vorgenannten Angaben zur Ausführbarkeit des mehrstufigen Gesamtverfahrens, umfassend die Herstellung der Verbindungen der Formel (4) in einem Flussreaktor, die Umsetzung der Verbindungen der Formel (4) zu Oxindolen der Formel (7-1) sowie deren anschließende Arylierung, Sulfonylierung, Oxidation und Alkylierung, die Eignung von Verbindungen der Formel (4) sowie die Eignung von Oxindole der Formeln (7-1), (6-1), (5-1), (2-1) und Verbindungen der Formel (1-1) zur Herstellung von Pflanzenschutzmitteln der Formel (4-1) belegt.

### erfolgt.

Besonders bevorzugte Imidazol-Basen für die Durchführung der Sulfonylierung sind 1-Methyl-1 H-Imidazol, 1-Butyl-1 H-Imidazol oder 1-Benzyl-1 H-Imidazol, die einzeln oder im Gemisch eingesetzt werden können, wobei die Verwendung von 1-Methyl-1 H-Imidazol ganz besonders bevorzugt ist.
- Oxidative Ringöffnung von N-sulfonylsubstituierten 3-Triazinyloxindolen (2-1) zu 2-(Triazinylcarbonyl)sulfonaniliden (1-1). Dieses Verfahren ist im technischen Maßstab möglich und ist Gegenstand der Patentanmeldung mit dem Aktenzeichen PCT/EP2011/073287.
- Alkylierung von 2-(Triazinylcarbonyl)sulfonaniliden (1-1) zu N-Alkyl-N-[2-(1,3,5-triazin-2-ylcarbonyl)phenyl]alkansulfonamiden (4-1). Dieses Verfahren ist in der Patentanmeldung WO 2006/008159 A1 beschrieben. Im Hinblick auf die Ausführbarkeit der Alkylierung wird hier auf den Inhalt der Patentanmeldung WO 2006/008159 A1 Bezug genommen.

Die Alkylierung kann mit gängigen Alkylierungsmitteln erfolgen. Im Falle einer Methylierung wird bevorzugt Dimethylsulfat eingesetzt.

Die herbizide Wirkung (siehe WO 2007/031208 A2) und fungizide Wirkung (siehe WO 2006/008159 A1) von N-Alkyl-N-[2-(1,3,5-triazin-2-ylcarbonyl)phenyl]alkansulfon-amiden der Formel (4-1) ist seit längerem bekannt.

Somit wird durch die vorgenannten Angaben zur Ausführbarkeit des mehrstufigen Gesamtverfahrens, umfassend die Herstellung der Verbindungen der Formel (4) in einem Flussreaktor, die Umsetzung der Verbindungen der Formel (4) zu Oxindolen der Formel (7-1) sowie deren anschließende Arylierung, Sulfonylierung, Oxidation und Alkylierung, die Eignung von Verbindungen der Formel (4) sowie die Eignung von Oxindolen der Formeln (7-1), (6-1), (5-1), (2-1) und Verbindungen der Formel (1-1) zur Herstellung von Pflanzenschutzmitteln der Formel (4-1) belegt.

### Beispiele

Das folgende Beispiel erläutert das erfindungsgemäße Verfahren näher, ohne dasselbe zu beschränken.

In den Erläuterungen des Beispiels beziehen sich Mengenangaben auf das Gewicht, sofern nichts anderes speziell definiert ist (in der Beschreibung wurde hierfür analog Gew.% = Gewichtsprozent verwendet). Für Maßeinheiten, physikalische Größen und ähnliches werden übliche Abkürzungen verwendet, beispielsweise h = Stunde(n), mpt. = Schmelzpunkt (Smp.), l = Liter, ml = Milliliter, g = Gramm, min = Minute(n), *in vacuo* = "im Vakuum" = unter reduziertem Druck, d. Th. = Prozent Ausbeute nach der Theorie.

### Synthesebeispiel 1:

### Kontinuierliche Durchführung einer Gassman Reaktion unter Verwendung der Stickstoffbase Chinolin

Das untenstehende Schema 6 zeigt den Aufbau eines Flussreaktors, der sich für die Ausführung des erfindungsgemäßen Verfahrens eignet.

In die Vorlage 1 (VO1) des Flussreaktors wird eine Lösung aus 7,5 Massenteilen 2-Fluoranilin (2-FA), 1,66 Massenteilen Chinolin (CL) und 90,84 Massenteilen Dichlormethan (DCM) gefüllt. Eine Lösung aus 7,5 Massenteilen Sulfurylchlorid (SO₂Cl₂; im Schema 6: SO2Cl2) und 92,5 Massenteilen Dichlormethan wird in die Vorlage 2 (VO2) des Flussreaktors gefüllt. Eine Lösung aus 7,96 Massenteilen Methylmethylthioacetat (MMTA), 4,88 Massenteilen Chinolin und 87,16 Massenteilen Dichlormethan wird in Vorlage 3 (VO3) des Flussreaktors gefüllt. In die Vorlage 4 (VO4) des Flussreaktors wird reines Chinolin gefüllt.

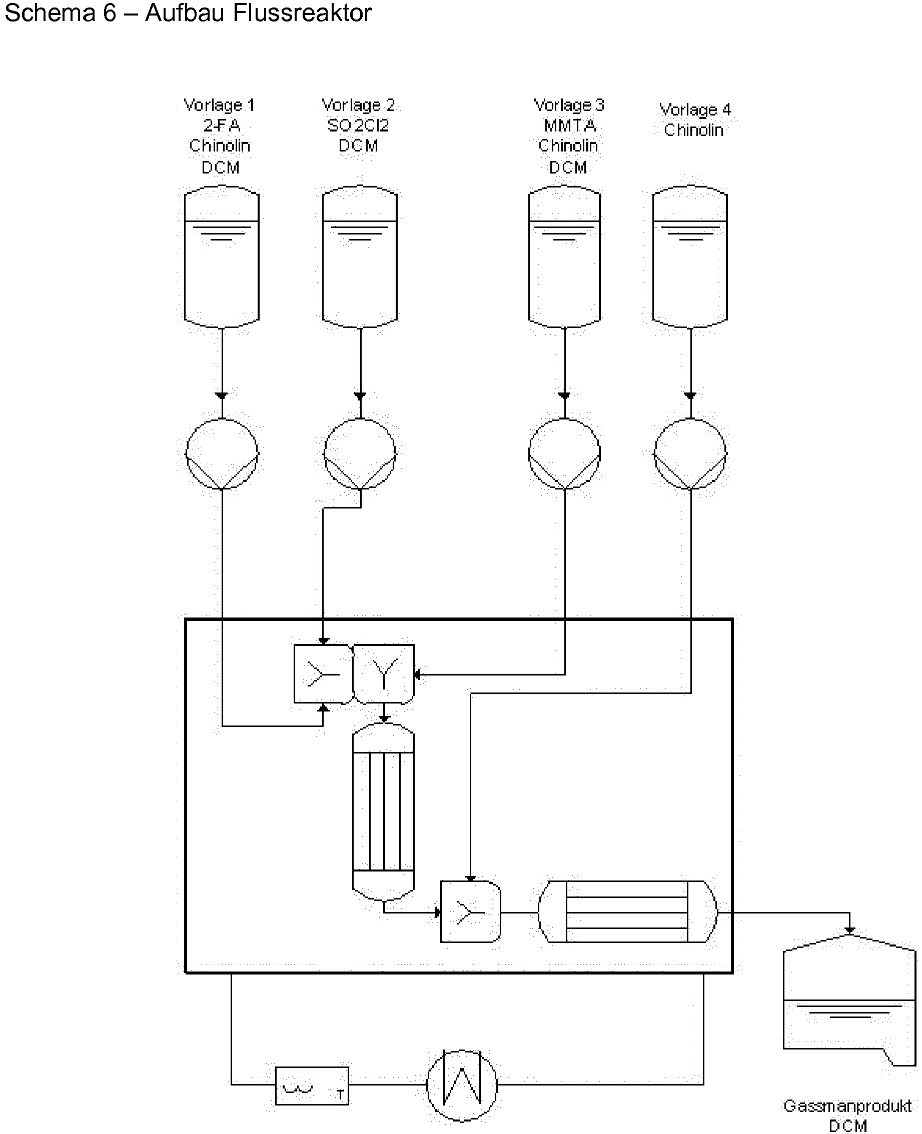

Die einzelnen Komponenten des Flussreaktors sowie die bei der kontinuierlich geführten Reaktion eingesetzten Edukte und Lösungsmittel werden durch die nachfolgend erläuterten Abkürzungen bezeichnet:
- - VO: Vorlage/Vorlagekammer
- - VG: Vorlagengemisch
- - M: Mischkammer
- - VW: Verweilelement
- - SG: Sammelgefäß

- - 2-FA: 2-Fluoranilin
- - CL: Chinolin
- - DCM: Dichlormethan
- - MMTA: Methylmethylthioacetat
- - SO2Cl2: Sulfonylchlorid (SO₂Cl₂)

Aus den Vorlagen 1 und 2 werden die angesetzten Substratlösungen über Vortemperierstrecken auf die Reaktionstemperatur (-40°C) abgekühlt und in einem ersten statischen Mischkammer (M1) mit einem Volumen von 0,3 cm³ zur Reaktion gebracht. Die Förderleistung der Pumpen wird dabei so gewählt, dass eine Verweilzeit von 0,16 Sekunden erreicht wird und ein stöchiometrisches Verhältnis von 2-Fluoranilin zu Sulfurylchlorid von 1,05 vorliegt. Das Reaktionsgemisch verlässt den ersten statischen Mischkammer (M1) und fließt direkt in eine zweite statische Mischkammer (M2) mit 0,6 cm³ Volumen.

In dem zweiten statischen Mischkammer (M2) wird die Reaktionslösung mit dem auf Reaktionstemperatur gebrachten Substratstrom, bestehend aus Methylmethylthioacetat, Chinolin und Dichlormethan aus Vorlage 3 zur Reaktion gebracht. Die Förderleistung der Pumpe wurde dabei so gewählt, dass eine Verweilzeit von 0,22 Sekunden erreicht wird und ein stöchiometrisches Verhältnis von Methylmethylthioacetat zu Sulfurylchlorid von 1,05 vorliegt.

Das Reaktionsgemisch verlässt die zweite Mischkammer und fließt in ein erstes Verweilelement (VW1) mit 90,84 cm³ Volumen und einer Verweilzeit von 32,6 Sekunden. An das Verweilelement VW1 ist ein weiterer dritter statischer Mischkammer (M3) mit einem Volumen von 0,3 cm³ angeschlossen. Im dritten statischen Mischkammer (M3) wird die Reaktionslösung mit dem auf Reaktionstemperatur gebrachten Chinolinstrom aus Vorlage 4 (VO4) zur Reaktion gebracht. Die Förderleistung der Pumpe wurde dabei so gewählt, dass eine Verweilzeit von 0,15 Sekunden erreicht wird und ein stöchiometrisches Verhältnis von Sulfurylchlorid zu Chinolin von 1,00 vorliegt.

Das Reaktionsgemisch verlässt die dritte Mischkammer und fließt in ein zweites Verweilelement (VW2) mit einem Volumen von 5,7 cm³ und einer Verweilzeit von 2 Sekunden. Das Reaktionsgemisch wird anschließend in einem Sammelgefäß (SG 1) aufgefangen. Die Reaktion wird regelmäßig per HPLC verfolgt.

Das Reaktionsgemisch wird bei 0°C in eine 0,4 N HCl geleitet. Die hellgelbe organische Phase wird von der wässrigen Phase getrennt. Die organische Phase wird mit 0,4 N HCl extrahiert, so dass das Produkt 4 als Lösung in Dichlormethan vorliegt.

Anschließend wird methanolische HCl zugesetzt und die Reaktionsmischung für 5 Stunden bei RT gerührt. Die Reaktionslösung wird bis 26 mbar bei 40°C aufkonzentriert. Der Rückstand wird mit Butylacetat verdünnt und mit n-Heptan versetzt. Nach 4 h Rühren der Reaktionsmischung bei RT ist die Kristallisation abgeschlossen. Das Produkt wird abfiltriert und zweifach mit n-Heptan gewaschen. Das sich durch die vorgenannte abschließende Behandlung ergebende Folgeprodukt der Formel (7-1) konnte mit einer Gesamtausbeute von 78% isoliert werden.

## Patentansprüche

1. Verfahren zur kontinuierlichen Herstellung von Verbindungen der Formel (4) 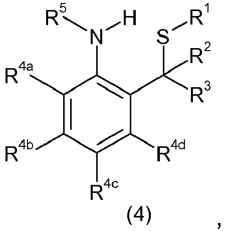 worin
R¹ für (C₁-C₆) Alkyl, substituiertes Alkyl, Aryl oder substituiertes Aryl steht,
R² für einen elektronenziehenden oder für einen aktivierenden Substituenten steht, der ausgewählt ist aus der Gruppe bestehend aus
- CN,
- NO₂,
- CO-R^{1'}, wobei R^{1'} wie R¹ definiert ist und R^{1'} gleich oder anders ist als R¹,
- CO-X, wobei X für OR^{1"}, SR^{1"} oder NR^{2'}R^{2"} steht, worin R^{1"} wie R¹ definiert ist und R^{1"} gleich oder anders ist als R¹, und worin
R^{2'} und R^{2"} unabhängig voneinander jeweils für H, (C₁-C₆) Alkyl, substituiertes (C₁-C₆) Alkyl, Aryl oder substituiertes Aryl stehen, oder R^{2'} und R^{2"} einen Ring bilden,
- SO(n)-R^{1"'}, worin R^{1"'} wie R¹ definiert ist, wobei R^{1"'} jeweils gleich oder anders ist als R¹ und wobei n 0, 1, oder 2 ist,
- Aryl, und
- Heteroaryl,
R³ für H, (C₁-C₆) Alkyl, substituiertes Alkyl, Aryl oder substituiertes Aryl steht,
R^{4a} bis R^{4d} unabhängig voneinander ausgewählt sind aus der Gruppe bestehend aus Wasserstoff, Fluor, Chlor, Brom, Iod, CN, NO₂ sowie aus
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
CO-X, wobei X für OR^{1"}, SR^{1"'} oder NR^{2'}R^{2"} steht, worin R^{1"} wie R¹ definiert ist und R^{1"} gleich oder anders ist als R¹, und worin R^{2'} und R^{2"} unabhängig voneinander jeweils für H, (C₁-C₆) Alkyl, substituiertes (C₁-C₆) Alkyl, Aryl oder substituiertes Aryl stehen, oder R^{2'} und R^{2"} alternativ einen Ring bilden,
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R⁵ für H, (C₁-C₆) Alkyl, substituiertes (C₁-C₆) Alkyl, Aryl oder substituiertes Aryl steht,
bei dem in einem Flussreaktor in Gegenwart eines organischen Lösungsmittels als Ed u kte
- ein Anilin (Q) 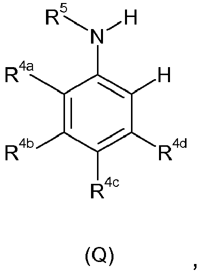 worin
R⁴, n und R⁵ wie für Verbindungen der Formel (4) definiert sind, und
- ein Thioether (W) 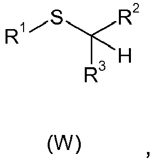
worin
R¹, R² und R³ wie für Verbindungen der Formel (4) definiert sind,
eingesetzt werden, wobei die Edukte der Formeln (Q) und (W) in Gegenwart
- eines Chlorierungsmittels, und
- mindestens einer Stickstoff-Base, die keine NH-Gruppe aufweist, umgesetzt werden.

2. Verfahren zur Herstellung von Verbindungen der Formel (4) in einem Flussreaktor nach Anspruch 1, wobei die Reaktionstemperatur in dem Bereich zwischen - 65° C und 0° C liegt.

3. Verfahren zur Herstellung von Verbindungen der Formel (4) in einem Flussreaktor nach Anspruch 2, wobei die Reaktionstemperatur in dem Bereich zwischen - 55° C und -10° C oder zwischen - 45° C und - 20° C liegt.

4. Verfahren zur Herstellung von Verbindungen der Formel (4) in einem Flussreaktor nach einem der Ansprüche 1 bis 3, wobei die Stickstoff-Base, bzw. die Stickstoff-Basen, ausgewählt sind aus der Gruppe bestehend aus
- tertiären Aminen N(R⁶⁰)₃ (P), worin die Reste R⁶⁰ ausgewählt sind aus der Gruppe der substituierten oder unsubstituierten1 C₁-C₁₈ Alkyl- und substituierten oder unsubstituierten (C₁-C₆)Aryl-Reste,
- cyclischen tertiären Aminen,
- substituierte und unsubstituierte Pyridine, und
- benzoanellierten Ringsysteme von substituierten oder unsubstituierten Pyridinen.

5. Verfahren zur Herstellung von Verbindungen der Formel (4) in einem Flussreaktor nach Anspruch 4, wobei mindestens eine der Stickstoff-Basen ein tertiäres Amin N(R⁶⁰)₃ (P) ist, worin die Reste R⁶⁰ jeweils für ein substituiertes oder unsubstituiertes C₁-C₁₈ Alkyl stehen, wobei mindestens einer der Reste R⁶⁰ eine Kettenlänge von mindestens C₆-C₁₈ aufweist oder die Reste R⁶ zusammengenommen eine Gesamtkettenlänge von mindestens C₆-C₁₈ aufweisen.

6. Verfahren zur Herstellung von Verbindungen der Formel (4) in einem Flussreaktor nach einem der Ansprüche 1 bis 5, wobei die Stickstoff-Base ausgewählt wird aus der Gruppe bestehend aus
- Tributylamin,
- 2-Methyl-5-Ethyl-Pyridin, und
- unsubstituiertes Chinolin.

7. Verfahren zur Herstellung von Verbindungen der Formel (4) in einem Flussreaktor nach einem der Ansprüche 1 bis 5, wobei ein Gemisch bestehend aus mindestens zwei der Stickstoff-Basen aus der Gruppe bestehend aus
- Tributylamin,
- 2-Methyl-5-Ethyl-Pyridin, und
- unsubstituiertes Chinolin
eingesetzt wird.

8. Verfahren zur Herstellung von Verbindungen der Formel (4) in einem Flussreaktor nach einem der Ansprüche 1 bis 7, wobei die eingesetzten Lösungsmittel ausgewählt werden aus der Gruppe bestehend aus
- Chloralkane,
- Aromaten,
- Haloaromaten,
- substituierte Aromaten, und
- eine Mischung, die eines oder mehrerer der vorgenannten unpolaren anorganischen Lösungsmittel enthält.

9. Verfahren zur Herstellung von Verbindungen der Formel (4) in einem Flussreaktor nach einem der Ansprüche 1 bis 8, wobei das Chlorierungsmittel ausgewählt ist aus der Gruppe bestehend aus
- tert-Butylhypochlorit,
- Sulfurylchlorid, oder
- einer Mischung aus tert-Butylhypochlorit und Sulfurylchlorid.

10. Verfahren zur Herstellung von Verbindungen der Formel (4) in einem Flussreaktor nach einem der Ansprüche 1 bis 9, wobei mindestens eines der beiden Edukte der Formel (Q) und der Formel (W) noch vor Zugabe des Chlorierungsmittels
- in einem organischen Lösungsmittel gelöst wird, und
- mit mindestens einer Stickstoff-Base, die keine NH-Gruppe aufweist und in dem gewählten organischen Lösungsmittel bei der jeweiligen Reaktionstemperatur gelöst vorliegt, gemischt wird.

11. Verfahren zur Herstellung von Verbindungen der Formel (4) in einem Flussreaktor nach einem der Ansprüche 1 bis 10, wobei die Stickstoff-Base, bzw. ein verschiedener Stickstoff-Basen umfassendes Gemisch, die jeweils keine NH-Gruppe aufweisen, den verschiedenen zu einem Flussreaktor gehörenden Vorlagekammern (VO) und/oder Mischkammern (M) und/oder Verweilstrecken (VW) mehrfach, d.h. wiederholt, zugeführt werden.

12. Verfahren zur Herstellung von Verbindungen der Formel (4) in einem Flussreaktor nach Anspruch 11, wobei in einer ersten Vorlagekammer (VO1) des Flussreaktors als Vorlage-Gemisch-1 (VG1), mindestens enthaltend
- ein Anilin (Q): 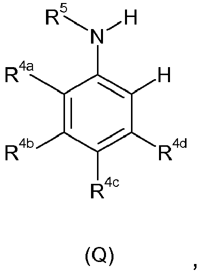 worin die Reste R⁴, n und R⁵ wie in Formel (4) definiert sind,
- ein organisches Lösungsmittel, in dem das Anilin (Q) gelöst ist, und
- mindestens eine Stickstoff-Base, die keine NH-Gruppe aufweist, vorgelegt wird, und
in einer zweiten Vorlagekammer (VO2) des Flussreaktors ein Vorlage-Gemisch-2 (VG2), mindestens enthaltend
- ein flüssiges Chlorierungsmittel und
- ein organisches Lösungsmittel, das dem in der ersten Vorlagekammer befindlichen Lösungsmittel entspricht,
vorgelegt wird, und
nachfolgend das Vorlage-Gemisch-1 (VG1) und das Vorlage-Gemisch-2 (VG2) in einer ersten Mischkammer (M1) bei einer Reaktionstemperatur im Bereich zwischen - 65°C und 0°C oder zwischen - 45°C und - 20°C gemischt werden, und
nachfolgend in einer zweiten Mischkammer (M2) das Reaktionsgemisch aus der ersten Mischkammer (M1) mit einem in einer dritten Vorlagekammer (VO3) eines Flussreaktors vorgelegten Vorlage-Gemisch-3 (VG3), mindestens enthaltend
- einen Thioether (W) 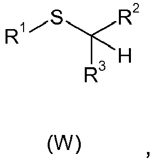 worin die Reste R¹, R² und R³ wie in Formel (4) definiert sind,
- ein organisches Lösungsmittel, das dem in der ersten Vorlagekammer vorgelegten Lösungsmittel entspricht, und
- eine Stickstoff-Base, die keine NH-Gruppe aufweist und der in der ersten Vorlagekammer vorgelegten Stickstoffbase entspricht.
vereinigt wird, und das so erhaltene Gemisch
- nachfolgend in einem ersten Verweil-Element-1 (VW-1) des Flussreaktors umgesetzt werden, und
- anschließend das in dem Verweil-Element-1 (VW-1) umgesetzte Gemisch in einer dritten Mischkammer (M3) des Flussreaktors erneut mit dem einer Stickstoff-Base, die keine NH-Gruppe aufweist, und der in der ersten Vorlagekammer vorgelegten Stickstoffbase entspricht, gemischt wird, und das so erhaltene Gemisch
- nachfolgend erneut in einem zweiten Verweil-Element-2 (VW-2) des Flussreaktors zu einer Verbindung der Formel (4) umgesetzt wird.

13. Mehrstufiges Verfahren zur Herstellung von N-Alkyl-N-[2-(1,3,5-triazin-2-ylcarbonyl)phenyl]alkansulfonamiden der Formel (4-1) 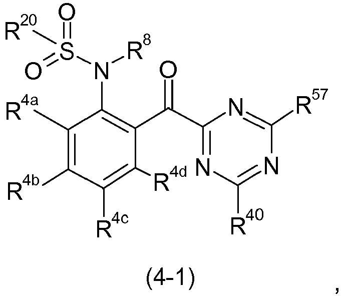 worin
R^{4a} bis R^{4d} unabhängig voneinander ausgewählt sind aus der Gruppe bestehend aus Wasserstoff, Fluor, Chlor, Brom, Iod, CN, NO₂ sowie aus
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkyl, wobei der Cycloalkylrest unsubstituiert ist oder durch einen oder mehrere Substituenten, ausgewählt aus der aus Fluor, Chlor, (C₁-C₄)-Alkyl oder (C₃-C₇)-Cycloalkyl oder (C₁-C₄)-Alkoxy bestehenden Gruppe, substituiert ist,
(C₁-C₆)-Alkoxy, wobei der Alkoxyrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₃-C₇)-Cycloalkoxy, wobei der Cycloalkoxyrest unsubstituiert ist oder durch einen oder mehrere Substituenten, ausgewählt aus der aus Fluor, Chlor, (C₁-C₄)-Alkyl oder (C₁-C₄)-Alkoxy bestehenden Gruppe, substituiert ist,
CO-X, wobei X für OR^{1"}, SR^{1"} oder NR^{2'}R^{2"} steht, worin R^{1"} wie R¹ definiert ist und R^{1"} gleich oder anders ist als R¹, und worin R^{2'} und R^{2"} unabhängig voneinander jeweils für H, (C₁-C₆) Alkyl, substituiertes (C₁-C₆) Alkyl, Aryl oder substituiertes Aryl stehen, oder R^{2'} und R²" alternativ einen Ring bilden,
Phenyl oder 1-Naphthyl oder 2-Naphthyl oder ein fünf- oder sechsgliedriger heteroaromatischer Ring mit 1 bis 2 Heteroatomen, wobei die Heteroatome unabhängig voneinander ausgewählt sind, aus der Gruppe bestehend aus O oder N und wobei der Aryl- oder Heteroarylrest unsubstituiert ist oder durch einen oder mehrere Substituenten, ausgewählt aus der aus Fluor, Chlor, Brom, Iod, (C₁-C₄)-Alkyl, (C₁-C₄)-Alkoxy oder (C₃-C₇)-Cycloalkyl oder (C₁-C₄)-Alkylthio bestehenden Gruppe, substituiert ist, und
R⁸ für
(C₁-C₆)-Alkyl, wobei der Alkylrest unsubstituiert ist oder ganz oder teilweise mit Fluor substituiert ist,
(C₁-C₆)-Cy_{C}loalkyl, (C₁-C₆)-Alkenyl oder (C₁-C₆)-Alkoxyalkyl, wobei jeder der genannten Reste unsubstituiert ist oder ganz oder teilweise mit Fluor substituiert ist,
steht,
R²⁰ für
(C₁-C₆)-Alkyl, wobei der Alkylrest unsubstituiert ist oder ganz oder teilweise mit Fluor substituiert ist, oder
(C₃-C₇)-Cycloalkyl, wobei der Cycloalkylrest unsubstituiert ist oder ganz oder teilweise mit Fluor substituiert ist, und
R⁴⁰ und R⁵⁷ unabhängig voneinander jeweils für
Wasserstoff,
(C₁-C₆)-Alkyl, wobei der Alkylrest unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
(C₁-C₆)-Alkoxy, wobei der Alkoxyrest verzweigt oder unverzweigt ist und unsubstituiert ist oder durch einen oder mehrere Substituenten, ausgewählt aus der aus Fluor, Chlor, (C₁-C₄)-Alkoxy oder (C₃-C₇)-Cycloalkyl bestehenden Gruppe, substituiert ist,
stehen,
ausgehend von einem 3-(Alkylsulfanyl)-1,3-dihydro-2H-indol-2-on der Formel (7-1), 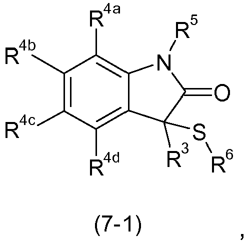 worin
R^{4a} bis R^{4d} wie für Formel (4) definiert sind,
R³ für Wasserstoff steht,
R⁵ für Wasserstoff steht, und
R⁶ wie R¹ für Formel (4) definiert ist, wobei
das 3-(Alkylsulfanyl)-1,3-dihydro-2H-indol-2-on der Formel (7-1) in einem ersten Schritt
durch
- Reduktion zu einem 1,3-Dihydro-2H-indol-2-on (6-1) 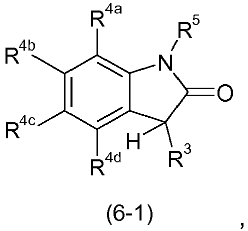 worin
R^{4a} bis R^{4d}, R³ und R⁵ wie für Formel (7-1) definiert sind,
umgewandelt wird, und das 1,3-Dihydro-2H-indol-2-on der Formel (6-1) 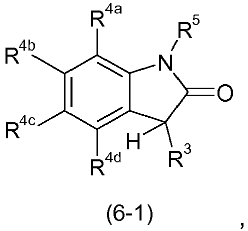 worin
R^{4a} bis R^{4d}, R³ und R⁵ wie für Formel (7-1) definiert sind,
in einem zweiten Schritt durch
- Arylierung zu einem triazinylsubstituierten Oxindol der Formel (5-1) 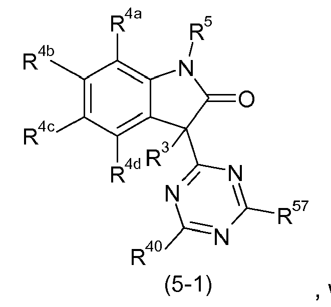 worin
R^{1a} bis R^{1d} und R⁴ und R⁵ wie für die Formel (4-1) und R³ und R⁷ wie für die Formel (6-1) definiert sind,
umgesetzt, und die Arylierungsprodukte der Formel (5-1) in einem dritten Schritt durch
- Sulfonylierung zu N-sulfonylsubstituierten 3-Triazinyloxindolen der Formel (2-1) 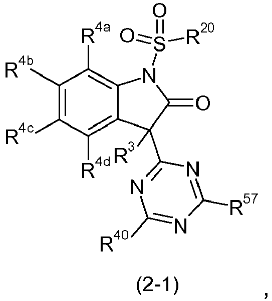 worin
R^{4a} bis R^{4d}, R²⁰ sowie R⁴⁰ und R⁵⁷wie in Formel (4-1) und R³ wie für die Formel (6-1)definiert sind,
umgesetzt, und die Sulfonylierungsprodukte der Formel (2-1) in einem vierten Schritt durch
- oxidative Ringöffnung zu einem 2-(Triazinylcarbonyl)sulfonanilid der Formel (1-1) 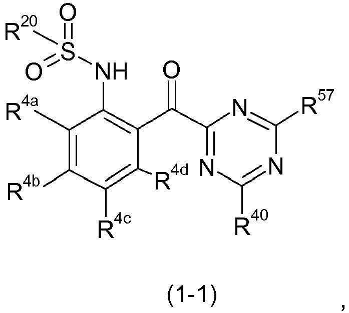 worin
R^{4a} bis R^{4d}, R²⁰ sowie R⁴⁰ und R⁵⁷ wie für Formel (4-1) definiert sind,
umgesetzt, und die Oxidationsprodukte der Formel (1-1) in einem fünften Schritt durch
- Alkylierung zu einem N-Alkyl-N-[2-(1,3,5-triazin-2-ylcarbonyl)phenyl] alkansulfonamid der Formel (4-1) 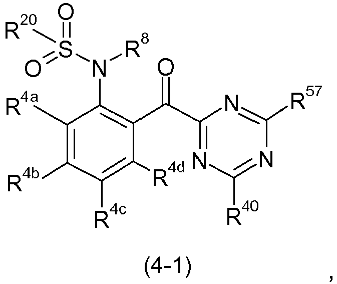 worin
R^{4a} bis R^{4d}, R²⁰ sowie R⁴⁰ und R⁵⁷ wie für Formel (4-1) definiert sind, umgesetzt, wobei
als Alkylierungsreagenz
- X- R⁸, worin X für Chlor, Brom, Iod oder OSO₂R⁹ steht, wobei R⁸ wie oben für Formel (4-1) definiert ist und R⁹ für (C₁-C₆) Alkyl, substituiertes Alkyl, Aryl oder substituiertes Aryl steht, oder
- (R⁸)₂SO₄, worin R⁸ wie oben für Formel (4-1) definiert ist, eingesetzt wird,
**dadurch gekennzeichnet, dass** das Edukt der Formel (7-1) bei kontinuierlicher Verfahrensführung in einem Flussreaktor nach einem der Ansprüche 1 bis 12 hergestellt wird.

## Claims

1. Process for continuously preparing compounds of the formula (4) 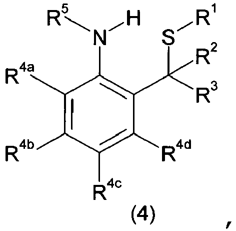 in which
R¹ is (C₁-C₆) alkyl, substituted alkyl, aryl or substituted aryl,
R² is an electron-withdrawing or activating substituent selected from the group consisting of
- CN,
- NO₂,
- CO-R^{1'}, where R^{1'} is as defined for R¹ and R^{1'} is the same as or different from R¹,
- CO-X, where X is OR^{1"}, SR^{1"} or NR^{2'}R^{2"}, in which
R^{1"} is as defined for R¹ and R^{1"} is the same as or different from R¹, and in which
R^{2'} and R^{2"} are each independently H, (C₁-C₆) alkyl, substituted (C₁-C₆) alkyl, aryl or substituted aryl, or R^{2'} and R^{2"} form a ring,
- SO(n)-R^{1"'}, where R^{1'"} is as defined for R¹, where each R^{1"'} is the same as or different from R¹ and where n is 0, 1 or 2,
- aryl, and
- heteroaryl,
R³ is H, (C₁-C₆) alkyl, substituted alkyl, aryl or substituted aryl,
R^{4a} to R^{4d} are each independently selected from the group consisting of hydrogen, fluorine, chlorine, bromine, iodine, CN, NO₂, and from
(C₁-C₆)-alkyl, where the alkyl radical is unsubstituted or substituted by one or more substituents selected from the group consisting of fluorine, chlorine, (C₁-C₄)-alkoxy and (C₃-C₇)-cycloalkyl,
(C₃-C₇)-cycloalkyl, where the cycloalkyl radical is unsubstituted or substituted by one or more substituents selected from the group conisisting of fluorine, chlorine, (C₁-C₄)-alkyl and (C₃-C₇)-cycloalkyl and (C₁-C₄)-alkoxy,
(C₁-C₆)-alkoxy, where the alkoxy radical is unsubstituted or substituted by one or more substituents selected from the group consisting of fluorine, chlorine, (C₁-C₄)-alkoxy and (C₃-C₇)-cycloalkyl,
(C₃-C₇)-cycloalkoxy, where the cycloalkoxy radical is unsubstituted or substituted by one or more substituents selected from the group consisting of fluorine, chlorine, (C₁-C₄)-alkyl and (C₁-C₄)-alkoxy,
CO-X, where X is OR^{1"}, SR^{1"} or NR^{2'}R^{2"}, in which R^{1"} is as defined for R¹ and R^{1"} is the same as or different from R¹, and in which R^{2'} and R^{2"} are each independently H, (C₁-C₆) alkyl, substituted (C₁-C₆) alkyl, aryl or substituted aryl, or R^{2'} and R^{2"} alternatively form a ring,
phenyl or 1-naphthyl or 2-naphthyl or a five- or six-membered heteroaromatic ring having 1 to 2 heteroatoms, where the heteroatoms are each independently selected from the group consisting of 0 and N, and where the aryl or heteroaryl radical is unsubstituted or substituted by one or more substituents selected from the group consisting of fluorine, chlorine, bromine, iodine, (C₁-C₄)-alkyl, (C₁-C₄) -alkoxy and (C₃-C₇) -cycloalkyl and (C₁-C₄) -alkylthio, and
R⁵ is H, (C₁-C₆) alkyl, substituted (C₁-C₆) alkyl, aryl or substituted aryl,
in which the reactants used in a flow reactor in the presence of an organic solvent are
- an aniline (Q) 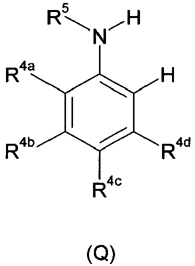
in which
R⁴, n and R⁵ are each as defined for compounds of the formula (4), and
- a thioether (W) 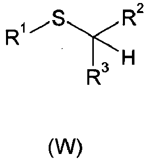
in which
R¹, R² and R³ are each as defined for compounds of the formula (4),
wherein the reactants of the formulae (Q) and (W) are converted in the presence of
- a chlorinating agent, and
- at least one nitrogen base having no NH group.

2. Process for preparing compounds of the formula (4) in a flow reactor according to Claim 1, wherein the reaction temperature is in the range between-65°C and 0°C.

3. Process for preparing compounds of the formula (4) in a flow reactor according to Claim 2, wherein the reaction temperature is in the range between-55°C and -10°C or between -45°C and -20°C.

4. Process for preparing compounds of the formula (4) in a flow reactor according to any of Claims 1 to 3, wherein the nitrogen base or nitrogen bases are selected from the group consisting of
- tertiary amines N(R⁶⁰)₃ (P), in which the R⁶⁰ radicals are selected from the group of the substituted or unsubstituted C₁-C₁₈ alkyl and substituted or unsubstituted (C₁-C₆) aryl radicals,
- cyclic tertiary amines,
- substituted and unsubstituted pyridines, and
- benzofused ring systems of substituted or unsubstituted pyridines.

5. Process for preparing compounds of the formula (4) in a flow reactor according to Claim 4, wherein at least one of the nitrogen bases is a tertiary amine N(R⁶⁰)₃ (P) in which the R⁶⁰ radicals are each a substituted or unsubstituted C₁-C₁₈ alkyl, where at least one of the R⁶⁰ radicals has a chain length of at least C₆-C₁₈ or the R⁶ radicals together have a total chain length of at least C₆-C₁₈.

6. Process for preparing compounds of the formula (4) in a flow reactor according to any of Claims 1 to 5, wherein the nitrogen base is selected from the group consisting of
- tributylamine,
- 2-methyl-5-ethylpyridine, and
- unsubstituted quinoline.

7. Process for preparing compounds of the formula (4) in a flow reactor according to any of Claims 1 to 5, wherein a mixture consisting of at least two of the nitrogen bases from the group consisting of
- tributylamine,
- 2-methyl-5-ethylpyridine and
- unsubstituted quinoline is used.

8. Process for preparing compounds of the formula (4) in a flow reactor according to any of Claims 1 to 7, wherein the solvents used are selected from the group consisting of
- chloroalkanes,
- aromatics,
- haloaromatics,
- substituted aromatics, and
- a mixture comprising one or more of the aforementioned nonpolar inorganic solvents.

9. Process for preparing compounds of the formula (4) in a flow reactor according to any of Claims 1 to 8, wherein the chlorinating agent is selected from the group consisting of
- tert-butyl hypochlorite,
- sulphuryl chloride, or
- a mixture of tert-butyl hypochlorite and sulphuryl chloride.

10. Process for preparing compounds of the formula (4) in a flow reactor according to any of Claims 1 to 9, wherein at least one of the two reactants of the formula (Q) and of the formula (W), before addition of the chlorinating agent,
- is dissolved in an organic solvent, and
- is mixed with at least one nitrogen base not having an NH group and present dissolved in the selected organic solvent at the respective reaction temperature.

11. Process for preparing compounds of the formula (4) in a flow reactor according to any of Claims 1 to 10, wherein the nitrogen base or a mixture comprising various nitrogen bases of which none have an NH group are supplied more than once, i.e. repeatedly, to the various reservoir chambers (RC) and/or mixing chambers (M) and/or dwell zones (DZ) which form part of a flow reactor.

12. Process for preparing compounds of the formula (4) in a flow reactor according to Claim 11, wherein a first reservoir chamber (RC1) of the flow reactor is initially charged with a reservoir mixture 1 (RM1) at least comprising
- an aniline (Q): 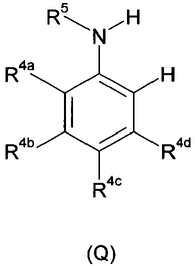 in which the R⁴ radicals, n and R⁵ radicals are each as defined in formula (4),
- an organic solvent in which the aniline (Q) is dissolved, and
- at least one nitrogen base not having an NH group,
and
a second reservoir chamber (RC2) of the flow reactor is initially charged with a reservoir mixture 2 (RM2) at least comprising
- a liquid chlorinating agent and
- an organic solvent corresponding to the solvent present in the first reservoir chamber,
and
then reservoir mixture 1 (RM1) and reservoir mixture 2 (RM2) are mixed in a first mixing chamber (M1) at a reaction temperature in the range between -65°C and 0°C or between -45°C and -20°C, and
then the reaction mixture from the first mixing chamber (M1) is combined in a second mixing chamber (M2) with a reservoir mixture 3 (RM3) initially charged in a third reservoir chamber (RC3) of a flow reactor, at least comprising
- a thioether (W) 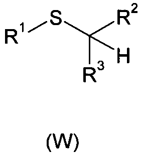 in which the R¹, R² and R³ radicals are each as defined in formula (4),
- an organic solvent corresponding to the solvent initially
charged in the first reservoir chamber, and
- a nitrogen base which does not have an NH group and corresponds to the nitrogen base initially charged in the first reservoir chamber,
and the mixture thus obtained
- is then converted in a first dwell element 1 (DE-1) of the flow reactor, and
- then the mixture converted in the dwell element 1 (DE-1) is mixed again in a third mixing chamber (M3) of the flow reactor with that nitrogen base which does not have an NH group and corresponds to the nitrogen base initially charged in the first reservoir chamber, and the mixture thus obtained
- is then converted again in a second dwell element 2 (DE-2) of the flow reactor to give a compound of the formula (4).

13. Multistage process for preparing N-alkyl-N-[2-(1,3,5-triazin-2-ylcarbonyl)-phenyl]alkanesulphonamides of the formula (4-1) 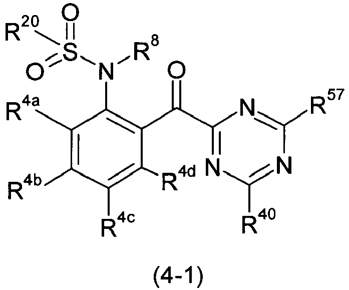 in which
R^{4a} to R^{4d} are each independently selected from the group consisting of hydrogen, fluorine, chlorine, bromine, iodine, CN, NO₂, and from
(C₁-C₆)-alkyl, where the alkyl radical is unsubstituted or substituted by one or more substituents selected from the group consisting of fluorine, chlorine, (C₁-C₄)-alkoxy and (C₃-C₇)-cycloalkyl,
(C₃-C₇)-cycloalkyl, where the cycloalkyl radical is unsubstituted or substituted by one or more substituents selected from the group conisisting of fluorine, chlorine, (C₁-C₄)-alkyl and (C₃-C₇)-cycloalkyl and (C₁-C₄)-alkoxy,
(C₁-C₆)-alkoxy, where the alkoxy radical is unsubstituted or substituted by one or more substituents selected from the group consisting of fluorine, chlorine, (C₁-C_{Q})-alkoxy and (C₃-C₇) -cycloalkyl, (C₃-C₇)-cycloalkoxy, where the cycloalkoxy radical is unsubstituted or substituted by one or more substituents selected from the group consisting of fluorine, chlorine, (C₁-C₄)-alkyl and (C₁-C₄)-alkoxy,
CO-X, where X is OR^{1"}, SR^{1"} or NR^{2'}R^{2"}, in which R^{1"} is as defined for R¹ and R^{1"} is the same as or different from R¹, and in which R^{2'} and R^{2"} are each independently H, (C₁-C₆) alkyl, substituted (C₁-C₆) alkyl, aryl or substituted aryl, or R^{2'} and R^{2"} alternatively form a ring,
phenyl or 1-naphthyl or 2-naphthyl or a five- or six-membered heteroaromatic ring having 1 to 2 heteroatoms, where the heteroatoms are each independently selected from the group consisting of 0 and N, and where the aryl or heteroaryl radical is unsubstituted or substituted by one or more substituents selected from the group consisting of fluorine, chlorine, bromine, iodine, (C₁-C₄)-alkyl, (C₁-C₄)-alkoxy and (C₃-C₇)-cycloalkyl and (C₁-C₄)-alkylthio, and
R8 is
(C₁-C₆)-alkyl, where the alkyl radical is unsubstituted or fully or partly substituted by fluorine,
(C₁-C₆)-cycloalkyl, (C₁-C₆)-alkenyl or (C₁-C₆)-alkoxyalkyl, where each of these radicals is unsubstituted or fully or partly substituted by fluorine,
R²⁰ is
(C₁-C₆)-alkyl, where the alkyl radical is unsubstituted or fully or partly substituted by fluorine, or
(C₃-C₇)-cycloalkyl, where the cycloalkyl radical is unsubstituted or fully or partly substituted by fluorine, and
R⁴⁰ and R⁵⁷ are each independently hydrogen,
(C₁-C₆)-alkyl, where the alkyl radical is unsubstituted or substituted by one or more substituents selected from the group consisting of fluorine, chlorine, (C₁-C₄)-alkoxy or (C₃-C₇)-cycloalkyl,
(C₁-C₆)-alkoxy, where the alkoxy radical is branched or unbranched and is unsubstituted or substituted by one or more substituents selected from the group consisting of fluorine, chlorine, (C₁-C₄) -alkoxy or (C₃-C₇)-cycloalkyl, proceeding from a 3-(alkylsulphanyl)-1,3-dihydro-2H-indol-2-one of the formula (7-1) 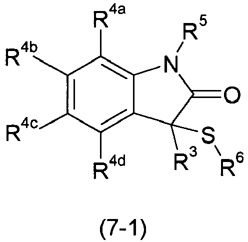 in which
R^{4a} to R^{4d} are each as defined for formula (4),
R³ is hydrogen,
R⁵ is hydrogen, and
R⁶ is as defined for R¹ for formula (4), wherein
the 3-(alkylsulphanyl)-1,3-dihydro-2H-indol-2-one of the formula (7-1) is converted in a first step by
- reduction to a 1,3-dihydro-2H-indol-2-one (6-1) 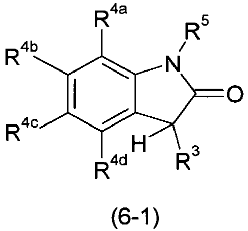 in which
R^{4a} to R^{4d}, R³ and R⁵ are each as defined for formula (7-1),
and the 1,3-dihydro-2H-indol-2-one of the formula (6-1) 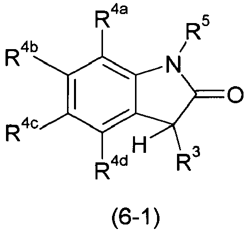 in which
R^{4a} to R^{4d}, R³ and R⁵ are each as defined for formula (7-1)
is converted in a second step by
- arylation to give a triazinyl-substituted oxindole of the formula (5-1) 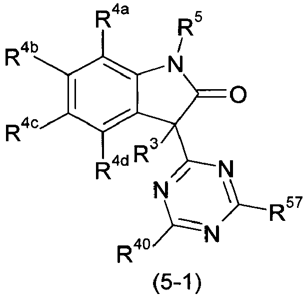 in which
R^{1a} to R^{1d} and R⁴ and R⁵ are each as defined for the formula (4-1) and R³ and R⁷ as defined for the formula (6-1),
and the arylation products of the formula (5-1) are converted in a
third step by
- sulphonylation to give N-sulphonyl-substituted 3-triazinyloxindoles of the formula (2-1) 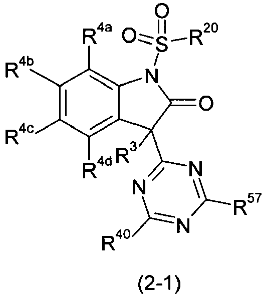 in which
R^{4a} to R^{4d}, R²⁰, and R⁴⁰ and R⁵⁷ are each as defined in formula (4-1) and R³ is as defined for the formula (6-1),
and the sulphonylation products of the formula (2-1) are converted in a
fourth step by
- oxidative ring opening to give a 2-(triazinylcarbonyl)sulphonanilide of the formula (1-1) 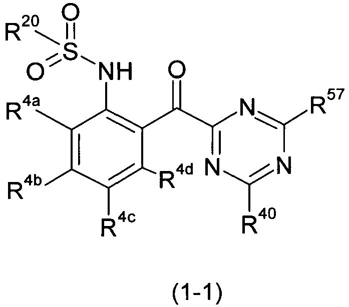 in which
R^{4a} to R^{4d}, R²⁰, and R⁴⁰ and R⁵⁷ are each as defined for formula (4-1),
and the oxidation products of the formula (1-1) are converted in a
fifth step by
- alkylation to give an N-alkyl-N-[2-(1,3,5-triazin-2-ylcarbonyl)phenyl]alkanesulphonamide of the formula (4-1) 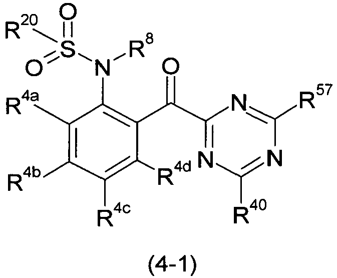 in which
R^{4a} to R^{4d}, R²⁰, and R⁴⁰ and R⁵⁷ are each as defined for formula (4-1),
wherein
the alkylating reagent used is
- X- R⁸, in which X is chlorine, bromine, iodine or OSO₂R⁹, where R⁸ is as defined above for formula (4-1) and R⁹ is (C₁-C₆) alkyl, substituted alkyl, aryl or substituted aryl, or
- (R⁸)₂SO₄, in which R⁸ is as defined above for formula (4-1),
**characterized in that** the reactant of the formula (7-1) is prepared in a continuous process regime in a flow reactor according to any of Claims 1 to 12.

## Revendications

1. Procédé de fabrication continue de composés de formule (4) 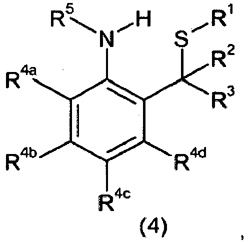 dans laquelle
R¹ représente alkyle en (C₁-C₆), alkyle substitué, aryle ou aryle substitué,
R² représente un substituant attracteur d'électrons ou activateur, qui est choisi dans le groupe constitué par
- CN,
- NO₂,
- CO-R^{1'}, R^{1'} étant défini tel que R¹ et R^{1'} étant identique ou différent de R¹,
- CO-X, X représentant OR^{1"}, SR^{1"} ou NR^{2'}R^{2"},
R^{1"} étant défini tel que R¹ et R^{1"} étant identique ou différent de R¹, et
R^{2'} et R^{2"} représentant chacun indépendamment l'un de l'autre H, alkyle en (C₁-C₆), alkyle en (C₁-C₆) substitué, aryle ou aryle substitué, ou R^{2'} et R^{2"} formant un cycle,
- SO(n)-R^{1"'}, R^{1"'} étant défini tel que R¹, R^{1'"} étant à chaque fois identique ou différent de R¹, et n représentant 0, 1 ou 2,
- aryle, et
- hétéroaryle,
R³ représente H, alkyle en (C₁-C₆), alkyle substitué, aryle ou aryle substitué,
R^{4a} à R^{4d} sont choisis indépendamment les uns des autres dans le groupe constitué par hydrogène, fluor, chlore, brome, iode, CN, NO₂ et
alkyle en (C₁-C₆), le radical alkyle étant non substitué ou substitué par un ou plusieurs substituants, choisis dans le groupe constitué par fluor, chlore, alcoxy en (C₁-C₄) ou cycloalkyle en (C₃-C₇),
cycloalkyle en (C₃-C₇), le radical cycloalkyle étant non substitué ou substitué par un ou plusieurs substituants choisis dans le groupe constitué par fluor, chlore, alkyle en (C₁-C₄) ou cycloalkyle en (C₃-C₇) ou alcoxy en (C₁-C₄),
alcoxy en (C₁-C₆), le radical alcoxy étant non substitué ou substitué par un ou plusieurs substituants choisis dans le groupe constitué par fluor, chlore, alcoxy en (C₁-C₄) ou cycloalkyle en (C₃-C₇),
cycloalcoxy en (C₃-C₇), le radical cycloalcoxy étant non substitué ou substitué par un ou plusieurs substituants choisis dans le groupe constitué par fluor, chlore, alkyle en (C₁-C₄) ou alcoxy en (C₁-C₄),
CO-X, X représentant OR^{1"}, SR^{1"} ou NR^{2'}R^{2"}, R^{1"} étant défini tel que R¹ et R^{1"} étant identique ou différent de R¹, et R^{2'} et R^{2"} représentant chacun indépendamment l'un de l'autre H, alkyle en (C₁-C₆), alkyle en (C₁-C₆) substitué, aryle ou aryle substitué, ou R^{2'} et R^{2"} formant en variante un cycle,
phényle ou 1-naphtyle ou 2-naphtyle ou un cycle hétéroaromatique à cinq ou six éléments contenant 1 à 2 hétéroatomes, les hétéroatomes étant choisis indépendamment les uns des autres dans le groupe constitué par 0 ou N, et le radical aryle ou hétéroaryle étant non substitué ou substitué par un ou plusieurs substituants choisis dans le groupe constitué par fluor, chlore, brome, iode, alkyle en (C₁-C₄), alcoxy en (C₁-C₄) ou cycloalkyle en (C₃-C₇) ou alkylthio en (C₁-C₄), et
R⁵ représente H, alkyle en (C₁-C₆), alkyle en (C₁-C₆) substitué, aryle ou aryle substitué,
selon lequel, dans un réacteur en écoulement en présence d'un solvant organique, en tant que réactifs,
sont utilisés
- une aniline (Q), 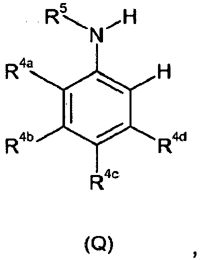
R⁴, n et R⁵ étant tels que définis pour les composés de formule (4), et
- un thioéther (W) 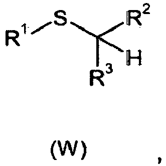
R¹, R² et R³ étant tels que définis pour les composés de formule (4),
les réactifs de formule (Q) et (W) étant mis en réaction en présence
- d'un agent de chloration et
- d'au moins une base azotée qui ne comprend pas de groupe NH.

2. Procédé de fabrication de composés de formule (4) dans un réacteur en écoulement selon la revendication 1, dans lequel la température de réaction se situe dans la plage comprise entre -65 °C et 0 °C.

3. Procédé de fabrication de composés de formule (4) dans un réacteur en écoulement selon la revendication 2, dans lequel la température de réaction se situe dans la plage comprise entre -55 °C et -10 °C ou entre - 45 °C et -20 °C.

4. Procédé de fabrication de composés de formule (4) dans un réacteur en écoulement selon l'une quelconque des revendications 1 à 3, dans lequel la base azotée ou les bases azotées sont choisies dans le groupe constitué par :
- les amines tertiaires N(R⁶⁰)₃(P), les radicaux R⁶⁰ étant choisis dans le groupe constitué par les radicaux alkyle en C₁-C₁₈ substitués ou non substitués et aryle en (C₁-C₆) substitués ou non substitués,
- les amines tertiaires cycliques,
- les pyridines substituées et non substituées, et
- les systèmes cycliques benzoannelés de pyridines substituées ou non substituées.

5. Procédé de fabrication de composés de formule (4) dans un réacteur en écoulement selon la revendication 4, dans lequel au moins une des bases azotées est une amine tertiaire N(R⁶⁰)₃(P), les radicaux R⁶⁰ représentant chacun un alkyle en C₁-C₁₈ substitué ou non substitué, au moins un des radicaux R⁶⁰ présentant une longueur de chaîne d'au moins C₆-C₁₈ ou les radicaux R⁶ pris ensemble présentant une longueur de chaîne totale d'au moins C₆-C₁₈.

6. Procédé de fabrication de composés de formule (4) dans un réacteur en écoulement selon l'une quelconque des revendications 1 à 5, dans lequel la base azotée est choisie dans le groupe constitué par :
- la tributylamine,
- la 2-méthyl-5-éthyl-pyridine et
- la quinoline non substituée.

7. Procédé de fabrication de composés de formule (4) dans un réacteur en écoulement selon l'une quelconque des revendications 1 à 5, dans lequel un mélange constitué par au moins deux des bases azotées du groupe constitué par
- la tributylamine,
- la 2-méthyl-5-éthyl-pyridine et
- la quinoline non substituée, est utilisé.

8. Procédé de fabrication de composés de formule (4) dans un réacteur en écoulement selon l'une quelconque des revendications 1 à 7, dans lequel les solvants utilisés sont choisis dans le groupe constitué par
- les chloroalcanes,
- les composés aromatiques,
- les composés haloaromatiques,
- les composés aromatiques substitués et
- un mélange qui contient un ou plusieurs des solvants inorganiques apolaires susmentionnés.

9. Procédé de fabrication de composés de formule (4) dans un réacteur en écoulement selon l'une quelconque des revendications 1 à 8, dans lequel l'agent de chloration est choisi dans le groupe constitué par :
- l'hypochlorite de tert-butyle,
- le chlorure de sulfuryle ou
- un mélange d'hypochlorite de tert-butyle et de chlorure de sulfuryle.

10. Procédé de fabrication de composés de formule (4) dans un réacteur en écoulement selon l'une quelconque des revendications 1 à 9, dans lequel au moins un des deux réactifs de formule (Q) et de formule (W) est, avant l'ajout de l'agent de chloration,
- dissous dans un solvant organique et
- mélangé avec au moins une base azotée qui ne comprend pas de groupe NH et qui se présente sous forme dissoute dans le solvant organique choisi à la température de réaction en question.

11. Procédé de fabrication de composés de formule (4) dans un réacteur en écoulement selon l'une quelconque des revendications 1 à 10, dans lequel la base azotée ou un mélange comprenant différentes bases azotées, qui ne comprennent chacune pas de groupe NH, est introduit à plusieurs reprises, c.-à-d. de manière répétée, dans les différentes chambres de préparation (VO) et/ou chambres de mélange (M) et/ou sections de séjour (VW) faisant partie d'un réacteur en écoulement.

12. Procédé de fabrication de composés de formule (4) dans un réacteur en écoulement selon la revendication 11, dans lequel, dans une première chambre de préparation (VO1) du réacteur en écoulement, un mélange de préparation 1 (VG1), contenant au moins une aniline (Q) : 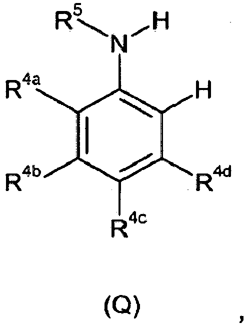 les radicaux R⁴, n et R⁵ étant tels que définis dans la formule (4),
- un solvant organique dans lequel l'aniline (Q) est dissoute, et
- au moins une base azotée qui ne comprend pas de groupe NH,
est chargé, et dans une deuxième chambre de préparation (VO2) du réacteur en écoulement, un mélange de préparation 2 (VG2), contenant au moins
- un agent de chloration liquide et
- un solvant organique qui correspond au solvant se trouvant dans la première chambre de préparation,
est chargé, puis le mélange de préparation 1 (VG1) et le mélange de préparation 2 (VG2) sont mélangés dans une première chambre de mélange (M1) à une température de réaction dans la plage comprise entre -65 °C et 0 °C ou entre-45 °C et -20 °C, puis dans une deuxième chambre de mélange (M2), le mélange réactionnel de la première chambre de mélange (M1) est réuni avec un mélange de préparation 3 (VG3) chargé dans une troisième chambre de préparation (VO3) d'un réacteur en écoulement, contenant au moins :
- un thioéther (W) 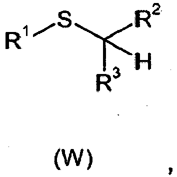
les radicaux R¹, R² et R³ étant tels que définis dans la formule (4),
- un solvant organique qui correspond au solvant chargé dans la première chambre de préparation, et
- une base azotée, qui ne comprend pas de groupe NH et qui correspond à la base azotée chargée dans la première chambre de préparation,
et le mélange ainsi obtenu
- est ensuite mis en réaction dans un premier élément de séjour 1 (VW-1) du réacteur en écoulement, puis
- le mélange mis en réaction dans l'élément de séjour 1 (VW-1) est de nouveau mélangé dans une troisième chambre de mélange (M3) du réacteur en écoulement avec la base azotée qui ne comprend pas de groupe NH et qui correspond à la base azotée chargée dans la première chambre de préparation, puis le mélange ainsi obtenu
- est de nouveau mis en réaction dans un deuxième élément de séjour 2 (VW-2) du réacteur en écoulement pour former un composé de formule (4).

13. Procédé à plusieurs étapes pour la fabrication de N-alkyl-N-[2-(1,3,5-triazin-2-ylcarbonyl)phényl]alcanesulfonamides de formule (4-1) 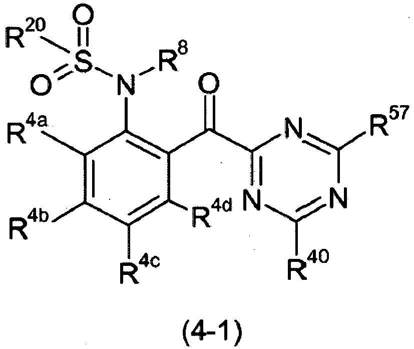 dans laquelle
R^{4a} à R^{4d} sont choisis indépendamment les uns des autres dans le groupe constitué par hydrogène, fluor, chlore, brome, iode, CN, NO₂ et
alkyle en (C₁-C₆), le radical alkyle étant non substitué ou substitué par un ou plusieurs substituants choisis dans le groupe constitué par fluor, chlore, alcoxy en (C₁-C₄) ou cycloalkyle en (C₃-C₇),
cycloalkyle en (C₃-C₇), le radical cycloalkyle étant non substitué ou substitué par un ou plusieurs substituants choisis dans le groupe constitué par fluor, chlore, alkyle en (C₁-C₄) ou cycloalkyle en (C₃-C₇) ou alcoxy en (C₁-C₄),
alcoxy en (C₁-C₆), le radical alcoxy étant non substitué ou substitué par un ou plusieurs substituants choisis dans le groupe constitué par fluor, chlore, alcoxy en (C₁-C₄) ou cycloalkyle en (C₃-C₇),
cycloalcoxy en (C₃-C₇), le radical cycloalcoxy étant non substitué ou substitué par un ou plusieurs substituants choisis dans le groupe constitué par fluor, chlore, alkyle en (C₁-C₄) ou alcoxy en (C₁-C₄),
CO-X, représentant OR^{1"}, SR^{1"} ou NR^{2'}R^{2"}, R^{1"} étant défini tel que R¹ et R^{1"} étant identique ou différent de R¹, et R^{2'} et R^{2"} représentant chacun indépendamment l'un de l'autre H, alkyle en (C₁-C₆), alkyle en (C₁-C₆) substitué, aryle ou aryle substitué, ou R^{2'} et R^{2"} formant en variante un cycle,
phényle ou 1-naphtyle ou 2-naphtyle ou un cycle hétéroaromatique à cinq ou six éléments contenant 1 à 2 hétéroatomes, les hétéroatomes étant choisis indépendamment les uns des autres dans le groupe constitué par O ou N, et le radical aryle ou hétéroaryle étant non substitué ou substitué par un ou plusieurs substituants choisis dans le groupe constitué par fluor, chlore, brome, iode, alkyle en (C₁-C₄), alcoxy en (C₁-C₄) ou cycloalkyle en (C₃-C₇) ou alkylthio en (C₁-C₄), et
R⁸ représente alkyle en (C₁-C₆), le radical alkyle étant non substitué ou substitué en totalité ou en partie avec fluor,
cycloalkyle en (C₁-C₆), alcényle en (C₁-C₆) ou alcoxyalkyle en (C₁-C₆), chacun des radicaux mentionnés étant non substitués ou substitués en totalité ou en partie avec fluor,
R²⁰ représente alkyle en (C₁-C₆), le radical alkyle étant non substitué ou substitué en totalité ou en partie avec fluor, ou
cycloalkyle en (C₃-C₇), le radical cycloalkyle étant non substitué ou substitué en totalité ou en partie avec fluor, et
R⁴⁰ et R⁵⁷ représentent chacun indépendamment l'un de l'autre
hydrogène,
alkyle en (C₁-C₆), le radical alkyle étant non substitué ou substitué par un ou plusieurs substituants choisis dans le groupe constitué par fluor, chlore, alcoxy en (C₁-C₄) ou cycloalkyle en (C₃-C₇),
alcoxy en (C₁-C₆), le radical alcoxy étant ramifié ou non ramifié, et non substitué ou substitué par un ou plusieurs substituants choisis dans le groupe constitué par fluor, chlore, alcoxy en (C₁-C₄) ou cycloalkyle en (C₃-C₇),
à partir d'une 3-(alkylsulfanyl)-1,3-dihydro-2H-indol-2-one de formule (7-1) 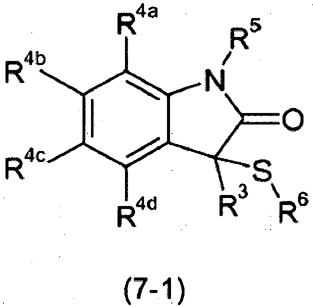 dans laquelle
R^{4a} à R^{4d} sont tels que définis pour la formule (4),
R³ représente l'hydrogène,
R⁵ représente l'hydrogène et
R⁶ est défini tel que R¹ pour la formule (4), la 3-(alkylsulfanyl)-1,3-dihydro-2H-indol-2-one de formule (7-1) étant transformée lors d'une première étape par
- réduction en une 1,3-dihydro-2H-indol-2-one (6-1) 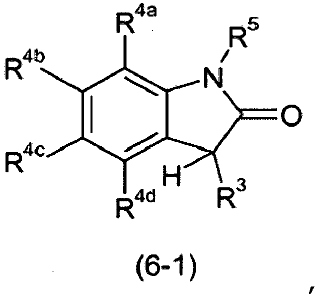 dans laquelle
R^{4a} à R^{4d}, R³ et R⁵ sont tels que définis pour la formule (7-1),
et la 1,3-dihydro-2H-indol-2-one de formule (6-1) 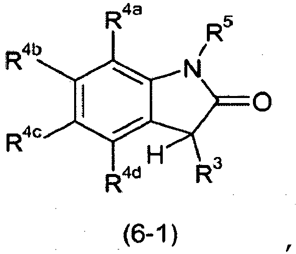 dans laquelle
R^{4a} à R^{4d}, R³ et R⁵ sont tels que définis pour la formule (7-1),
est mise en réaction lors d'une deuxième étape par
- arylation en un oxindole à substitution triazinyle de formule (5-1) 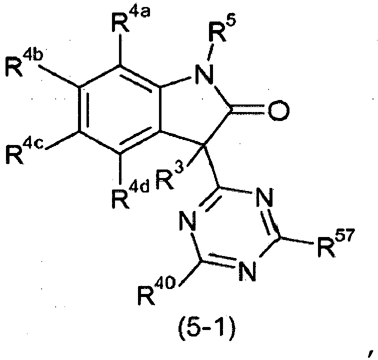 dans laquelle
R^{1a} à R^{1d} et R⁴ et R⁵ sont tels que définis pour la formule (4-1) et R³ et R⁷ sont tels que définis pour la formule (6-1),
et les produits d'arylation de formule (5-1) sont mis en réaction lors d'une troisième étape par
- sulfonation en 3-triazinyloxindoles à substitution N-sulfonyle de formule (2-1) 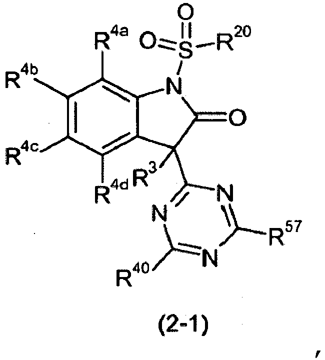 dans laquelle
R^{4a} à R^{4d}, R²⁰, ainsi que R⁴⁰ et R⁵⁷, sont tels que définis dans la formule (4-1), et R³ est tel que défini pour la formule (6-1),
et les produits de sulfonation de formule (2-1) sont mis en réaction lors d'une quatrième étape par
- ouverture de cycle oxydative pour former un 2-(triazinylcarbonyl)sulfonanilide de formule (1-1) 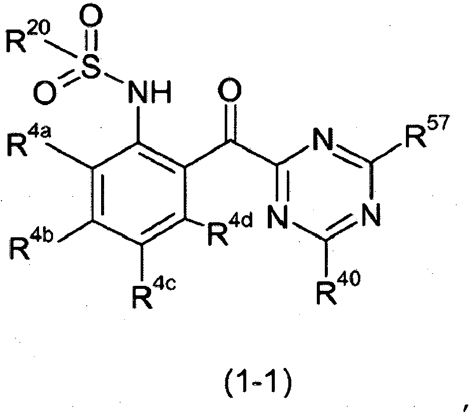 dans laquelle
R^{4a} à R^{4d}, R²⁰, ainsi que R⁴⁰ et R⁵⁷, sont tels que définis pour la formule (4-1),
et les produits d'oxydation de formule (1-1) sont mis en réaction lors d'une cinquième étape par
- alkylation en un N-alkyl-N-[2-(1,3,5-triazin-2-ylcarbonyl)phényl]alcanesulfonamide de formule (4-1) 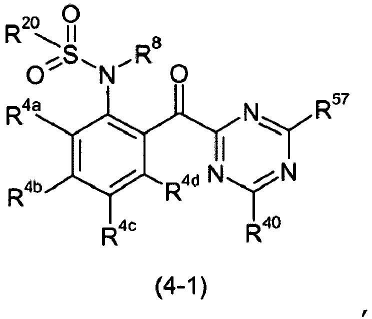 dans laquelle
R^{4a} à R^{4d}, R²⁰ ainsi que R⁴⁰ et R⁵⁷ sont tels que définis pour la formule (4-1),
en tant que réactif d'alkylation,
- X-R⁸, X représentant chlore, brome, iode ou OSO₂R⁹, R⁸ étant tel que défini précédemment pour la formule (4-1) et R⁹ représentant alkyle en (C₁-C₆), alkyle substitué, aryle ou aryle substitué, ou
- (R⁸)₂SO₄, R⁸ étant tel que défini précédemment pour la formule (4-1),
étant utilisé,
**caractérisé en ce que** le réactif de formule (7-1) est fabriqué par un procédé continu dans un réacteur en écoulement selon l'un quelconque des revendications 1 à 12.
